# EUROPEAN PATENT APPLICATION

(11) **EP 4 796 130 A1**
(43) Date of publication of application: **26.08.2026**
(21) Application number: 24880198.7
(22) Date of filing: 18.10.2024
(51) Int. Cl.: A61K 47/68, A61P 35/00, C07K 16/36

(54) **ANTI-TISSUE FACTOR ANTIBODY-DRUG CONJUGATE COMPRISING CAMPTOTHECIN DERIVATIVE**

(30) Priority: 19.10.2023 KR 20230140704
(71) Applicant: Celltrion, Inc., Incheon 22014 (KR)
(72) Inventor: YANG, Sungjae, Incheon 22014 (KR); KIM, Woogyum, Incheon 22014 (KR); CHO, Jinhwan, Incheon 22014 (KR); YOO, Byounggyu, Incheon 22014 (KR); HWANG, Boram, Incheon 22014 (KR); YOON, Joonsun, Incheon 22014 (KR); JUNG, Doo Young, Suwon-si, Gyeonggi-do 16506 (KR); CHO, Hyun Yong, Suwon-si, Gyeonggi-do 16506 (KR); LEE, Byeong Sung, Suwon-si, Gyeonggi-do 16506 (KR); CHUN, Young Hwa, Suwon-si, Gyeonggi-do 16506 (KR); KIM, Jongin, Incheon 22014 (KR); SEO, Jimin, Incheon 22014 (KR); JUNG, Myungho, Incheon 22014 (KR)
(74) Representative: Maiwald GmbH
(86) International application number: PCT/KR2024/015907
(87) International publication number: WO 2025/084861

(57) **Abstract**

The present invention provides an antibody-drug conjugate having a structure in which a compound according to chemical formula 1 or a pharmaceutically acceptable salt, stereoisomer, tautomer, prodrug, hydrate, solvate or isotope-labeled analog thereof is conjugated to an anti-TF antibody via a linker. The antibody-drug conjugate according to the present invention can selectively exhibit the effect of the drug compound of chemical formula 1 against cancer cells expressing tissue factors (TFs).

## Description

### Technical Field

The present invention relates to an anti-Tissue factor antibody-drug conjugate having a structure in which a compound according to Formula 1 is linked to an antibody via a linker.

In addition, the present invention relates to a method for treating or preventing cancer using the antibody-drug conjugate, and relates to a pharmaceutical composition for treating or preventing cancer including the antibody-drug conjugate as an active ingredient, a method for treating or preventing cancer including administering the same, and a use thereof.

### Background Art

Cancer is one of the most serious diseases, and according to the statistics from the World Health Organization (WHO), cancer incidence is increasing every year worldwide, thus requiring the development of new anticancer drugs in the pharmaceutical industry. In this regard, camptothecin (CPT), which is an alkaloid isolated from a plant of the family *Davidiaceae*, has been identified to have efficacy as a cytotoxic anticancer substance to inhibit topoisomerase, and camptothecin drugs, such as irinotecan (CPT-11), topotecan, 10-hydroxy camptothecin, belotecan (CKD-602), and exatecan have been developed domestically and internationally. In addition, a substance called DXd has been recently developed, and the development of new camptothecin derivatives is continuously progressing.

However, since cytotoxic anticancer agents lack cell selectivity, they attack normal cells as well as cancer cells, thereby causing unexpected side effects. Therefore, cytotoxic anticancer agents are often prepared as antibody-drug conjugates (ADCs) by conjugating antibodies that bind to specific target antigens on the surface of cancer cells with drugs that have cytotoxic effects.

Meanwhile, Tissue factor (TF), which is also known as thromboplastin, coagulation factor III, or CD142, is a protein present in subendothelial tissue, platelets, and leukocytes, which are required for the initiation of thrombin formation from prothrombin. Tissue factor (TF) is a cell surface receptor for the serine protease factor VIIa (FVIIa), which enables cells to initiate the blood coagulation cascade. Meanwhile, as Tissue factor (TF) expression has been observed in various types of cancer, including pancreatic cancer, cervical cancer, and skin cancer, it is attracting attention as a clinically important therapeutic target in anticancer treatment.

Accordingly, the development of antibody-drug conjugates that have high cytotoxicity and specifically act on cancer cells expressing Tissue factor (TF), by including camptothecin derivative compounds as drugs, is becoming a new challenge.

### Disclosure of Invention

### Technical Problem

Therefore, an object of the present invention is to provide an anti-TF antibody-drug conjugate, which includes a camptothecin derivative having excellent cytotoxicity activity and excellent target specificity for cells expressing Tissue factor (TF), and pharmaceutical uses thereof.

### Solution to Problem

In order to solve the above problems, the present invention provides an antibody-drug conjugate (ADC) having a structure, in which a compound according to Formula 1 or a pharmaceutically acceptable salt, a stereoisomer, a tautomer, a prodrug, a hydrate, a solvate, or an isotopically labeled analog thereof is linked to an anti-TF antibody via a linker.

Another aspect of the present invention provides a method for treating or preventing cancer, which includes contacting the antibody-drug conjugate with cancer cells.

A still another aspect of the present invention provides a pharmaceutical composition for treating or preventing cancer, which includes the antibody-drug conjugate as an active ingredient; a method for treating or preventing cancer including administering the same; and a use thereof.

### Advantageous Effects of Invention

The antibody-drug conjugate according to the present invention can exhibit the effect of a compound drug of Formula 1 in a target-specific manner on cells expressing Tissue factor (TF).

The compound of Formula 1, which is an active camptothecin derivative designed to bind to DDX5 protein and E3 ligase, may be one that is able to bind to E3 ligase as a molecular glue; and/or is able to kill target cells expressing DDX5 protein via a molecular glue mechanism of action (MoA); and/or has a mechanism of action (MoA) that degrades oncoprotein DDX5 along with the ability to inhibit type 1 topoisomerase.

The antibody-drug conjugate of the present invention can easily penetrate cell membranes due to the hydrophobicity of the compound of Formula 1, and when it enters cancer cells, the linker is specifically cleaved by a lysosomal protease highly expressed in tumors, thereby releasing the drug of Formula 1 and killing the cancer cells. The drug can then be released and cause a bystander effect that kills surrounding cancer cells. This allows the drug to exhibit an excellent killing effect not only on cancer cells that highly express Tissue factor (TF), but also on cancer cells that have low expression.

### Brief Description of Drawings

Fig. 1 shows the results of size exclusion chromatography (SEC) of the antibody-drug conjugate (ADC) of Example 3.
Fig. 2 shows the results of flow cytometry analysis for PE-mAb (12-1429-42) and APC-mAb (365205), which are commercially available monoclonal antibodies, in the process of selecting a cell line expressing Tissue factor (TF).
Fig. 3 shows the results of flow cytometry analysis for Tisotumab & AF647-F(ab)₂ anti-human IgG-F(ab)₂ (109-606-006) and Tivdak^{TM} (Lot:196169) & AF647-F(ab)₂ anti-human IgG-F(ab)₂ (109-606-006) in the process of selecting a cell line expressing Tissue factor (TF).
Fig. 4 shows the results of evaluation of *in-vitro* cell viability when ADC was treated in T47D (a breast cancer cell line).
Fig. 5 shows the results of evaluation of *in-vitro* cell viability when ADC was treated in MCF-7 (a breast cancer cell line).
Fig. 6 shows the results of evaluation of *in-vitro* cell viability when ADC was treated in KYSE-520 (an esophageal cancer cell line).
Fig. 7 shows the results of evaluation of *in-vitro* cell viability when ADC was treated in Caski (a cervical cancer cell line).
Fig. 8 shows the results of evaluation of *in-vitro* cell viability when ADC was treated in HPAF-II (a pancreatic cancer cell line).
Fig. 9 shows the results of evaluation of *in-vitro* cell viability when ADC was treated in A431 (a skin cancer cell line).
Fig. 10 shows the body weight changes in female BALB/c nude mice, in each group, where KYSE520 is transplanted.
Fig. 11 shows the tumor growth curve in female BALB/c nude mice, in each group, where KYSE520 is transplanted.
Fig. 12 shows the body weight changes in female CB17 SCID mice, in each group, where HPAFII is transplanted.
Fig. 13 shows the tumor growth curve in female CB17 SCID mice, in each group, where HPAFII is transplanted.

### Best Mode for Carrying out the Invention

The present invention will be described in more detail below.

The embodiments of the present invention may be modified in various different forms, and the scope of the present invention is not limited to the embodiments described below. Additionally, the embodiments of the present invention are provided to more completely explain the present invention to a person of ordinary knowledge in the art. Furthermore, in order to "include" a component throughout a specification means that, unless otherwise specifically stated, it may include other elements, rather than excluding other elements.

According to an aspect of the present invention, there is provided an antibody-drug conjugate (ADC) having a structure, in which a compound according to Formula 1 below or a pharmaceutically acceptable salt, a stereoisomer, a tautomer, a prodrug, a hydrate, a solvate, or an isotopically labeled analog thereof is linked to an anti-TF antibody via a linker: 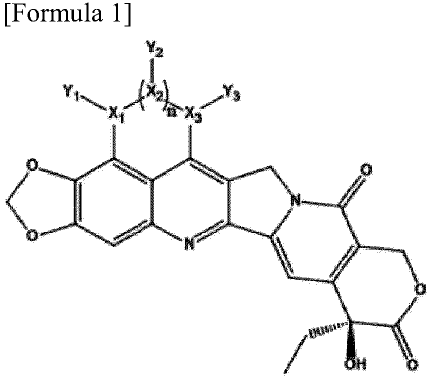 wherein:
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
n may be 0, 1, or 2; and
Y₁, Y₂, and Y₃ may each independently be hydrogen or a functional group including oxygen, nitrogen, phosphorus, or sulfur.

In this case, when X₂ is a single bond or double bond, n may be 0. More specifically, it may be a single bond or double bond linking X₁ and X₃.

In particular, non-limiting examples of the functional group including oxygen, nitrogen, phosphorus, or sulfur may be those which include a functional group selected from the group consisting of -CHO, -COOH, -NH₂, -SH, -CONH₂, -PO₃H, -PO₄H₂, - OPO₄H, -PO₂(OR¹)(OR²) (R¹, R² = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -SO₃H, -OSO₃H, -NO₂, - N₃, -NR₃OH(R=CₙH₂ₙ₊₁, 0≤n≤16), -NR₃⁺X⁻(R= CₙHₘ, 0≤n≤16, 0≤m≤ 34, X = OH, Cl, or Br), NR₄⁺X⁻(R= CₙHₘ, 0≤n≤16, 0≤m≤34, X = OH, Cl, or Br), -COSH, -COOCO-, - CORCO- (R = CₙHₘ, 0≤n≤3, 0≤m≤2l+1), -COOR, -CN, -N₃, -N₂, -NROH(R = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹NR²R³ (R¹, R², R³= CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), - CONHNR¹R² (R¹, R² = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹R²R³X' (R¹, R², R³ = CₛHₜNᵤO_{w}SₓP_{y}X_{z}, X = -F, -Cl, -Br, or -I, X' = F-, Cl-, Br-, or I-, 0≤s≤20, 0≤t≤2(s+u)+1, 0≤u≤2s, 0≤w≤2s, 0≤x≤2s, 0≤y≤2s, 0≤z≤2s), -OH, -O-, >C=O, -SS-, -SO-, -NO₂, -COX (X = F, Cl, Br, or I), -COOCO-, -CONH-, -CN, -SCOCH₃, -SCN, -NCS, -NCO, -OCN, -CN, -F, -Cl, -I, - Br, an epoxy group, -hydrazone, -ONO₂, -PO(OH)₂, -C=NNH₂, -HC=CH-, -C=C-, - C≡C-, and a hydrocarbon with two or more carbon atoms.

According to an aspect of the present invention, there is provided an antibody-drug conjugate (ADC) having a structure, in which a compound according to Formula 1 below or a pharmaceutically acceptable salt, a stereoisomer, a tautomer, a prodrug, a hydrate, a solvate, or an isotopically labeled analog thereof is linked to an anti-TF antibody via a linker:

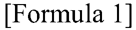

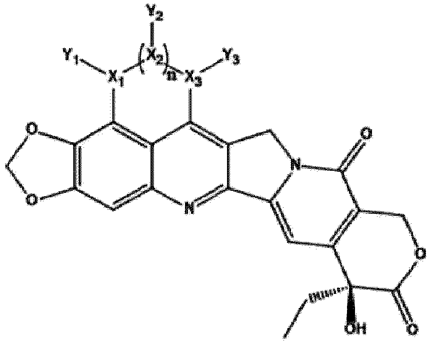

In particular,
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
X₁, (X₂)ₙ, and X₃ may together form a 6-membered or 7-membered ring, where n is 1 or 2; and
Y₁, Y₂, and Y₃ may each independently be hydrogen or a functional group including oxygen, nitrogen, phosphorus, or sulfur.

In particular, non-limiting examples of the functional group including oxygen, nitrogen, phosphorus, or sulfur may be those which include-CHO, -COOH, -NH₂, -SH, -CONH₂, -PO₃H, -PO₄H₂, -OPO₄H, -PO₂(OR¹)(OR²)(R¹, R² = CₛHₜNᵤO_{w}SₓP_{y}X_{z}, X = - F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), - SO₃H, -OSO₃H, -NO₂, -N₃, -NR₃OH (R = CₙH₂ₙ₊₁, 0≤n≤16), -NR₃⁺X⁻ (R= CₙHₘ, 0≤n≤16, 0≤m≤ 34, X = OH, Cl, or Br), NR₄⁺X⁻(R= CₙHₘ, 0≤n≤16, 0≤m≤34, X = OH, Cl, or Br), -COSH, -COOCO-, -CORCO- (R = CₙHₘ, 0≤n≤3, 0≤m≤2l+1), -COOR, - CN, -N₃, -N₂, -NROH(R = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹NR²R³(R¹, R², R³ = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -CONHNR¹R² (R¹, R² = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹R²R³X' (R¹, R², R³ = CₛHₜNᵤO_{w}SₓP_{y}X_{z}, X = -F, -Cl, -Br, or -I, X'= F-, Cl-, Br, or I-, 0≤s≤20, 0≤t≤2(s+u)+1, 0≤u≤2s, 0≤w≤2s, 0≤x≤2s, 0≤y≤2s, 0≤z≤2s), -OH, -O-, >C=O, -SS-, - SO-, -NO₂, -COX (X = F, Cl, Br, or I), -COOCO-, -CONH-, -CN, -SCOCH₃, -SCN, - NCS, -NCO, -OCN, -CN, -F, -Cl, -I, -Br, an epoxy group, -hydrazone, -ONO₂, - PO(OH)₂, -C=NNH₂, -HC=CH-, -C=C-, -C≡C-, and a hydrocarbon with two or more carbon atoms.

According to an aspect of the present invention, in Formula 1 above,
X₁, X₂, and X₃ may each independently be carbon;
n may be 1;
Y₁ and Y₂ may each independently be hydrogen; and
Y₃ may be hydrogen, or a functional group including oxygen, nitrogen, phosphorus, or sulfur.

In this case, non-limiting examples of the functional group including oxygen, nitrogen, phosphorus, or sulfur may be the same as the examples above.

According to an aspect of the present invention, in Formula 1 above,
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
n may be 0, 1, or 2; and
Y₁, Y₂, and Y₃ may each independently be hydrogen or a functional group including oxygen, nitrogen, phosphorus, or sulfur.

The functional group including nitrogen may be -NZ₁Z₂; and
Z₁ and Z₂ may each independently be one which includes a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be hydrogen, or a functional group including oxygen, nitrogen, phosphorus, or sulfur.

In this case, non-limiting examples of the functional group including oxygen, nitrogen, phosphorus, or sulfur may be the same as the examples above.

According to an aspect of the present invention, in Formula 1 above,
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
X₁, (X₂)ₙ, and X₃ may together form a 6-membered or 7-membered ring, where n is 1 or 2; and
Y₁, Y₂, and Y₃ may each independently be hydrogen or a functional group including oxygen, nitrogen, phosphorus, or sulfur.

The functional group including nitrogen may be -NZ₁Z₂; and
Z₁ and Z₂ may each independently be one which includes a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ and Z₂ may each independently be one which includes a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ and Z₂ may each independently be hydrogen, C(=O)H, C(=O)(C₁₋₃ alkyl), C(=O)(C₂₋₃ alkenyl), C(=O)NH₂, C(=O)NH(C₁₋₃ alkyl), or C(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ may be hydrogen, and Z₂ may be C(=O)(C₂₋₃ alkenyl) or C(=O)CR¹R²R³.

In this case, R¹, R², and R³ may each independently be hydrogen, hydroxyl, C₁₋₃ alkyl, C₃₋₅ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ may be hydrogen, and Z₂ may be C(=O)CR¹R²R³.

In particular, R¹, R², and R³ may each independently be hydrogen, hydroxyl, or C₁₋₃ alkyl.

According to an aspect of the present invention, the compound of Formula 1 above may be a compound represented by any one of Formulas 2 to 11 below, or a pharmaceutically acceptable salt, a stereoisomer, a tautomer, a prodrug, a hydrate, a solvate, or an isotopically labeled analog thereof.

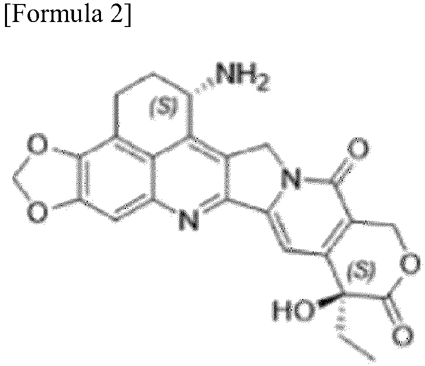

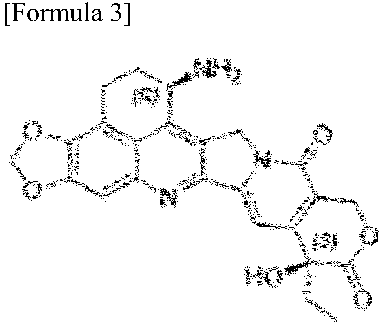

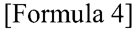

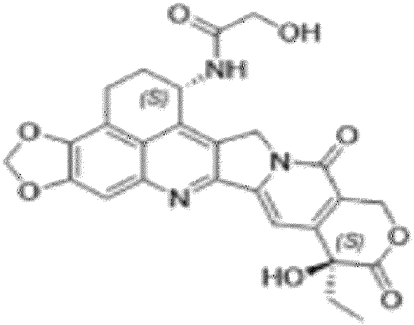

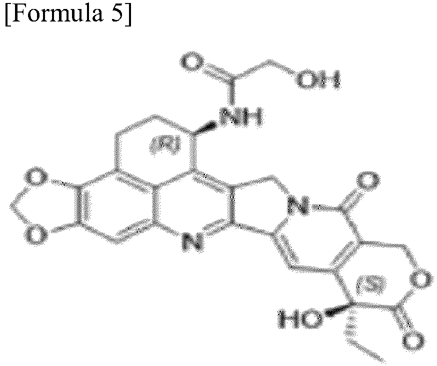

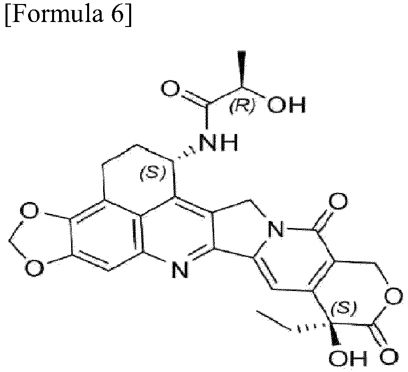

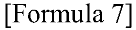

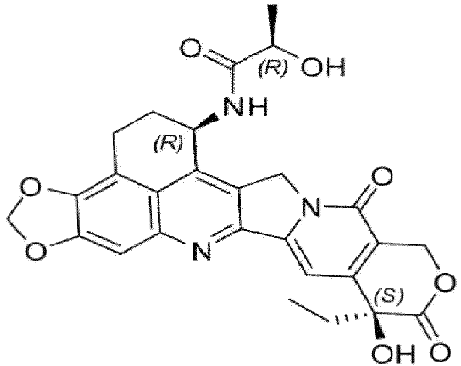

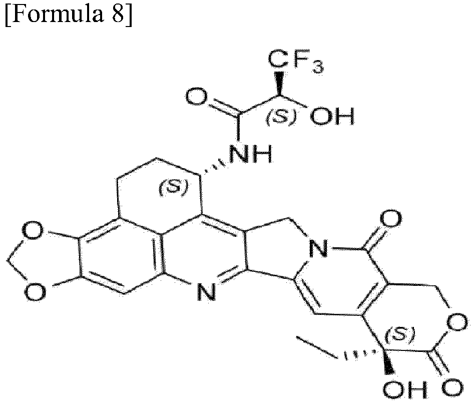

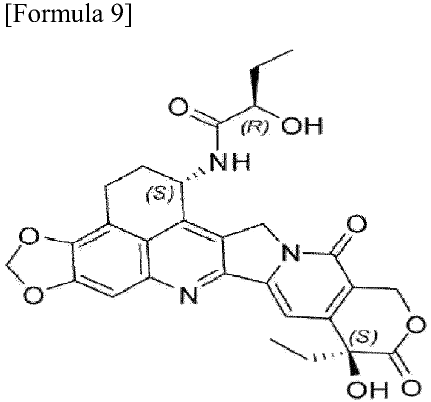

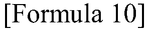

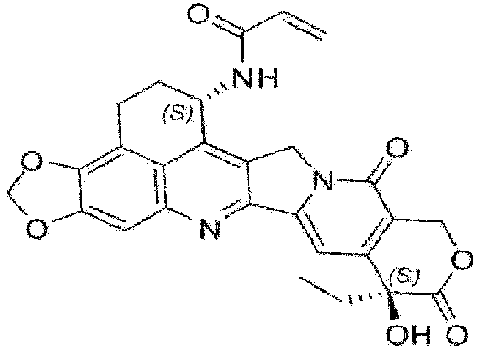

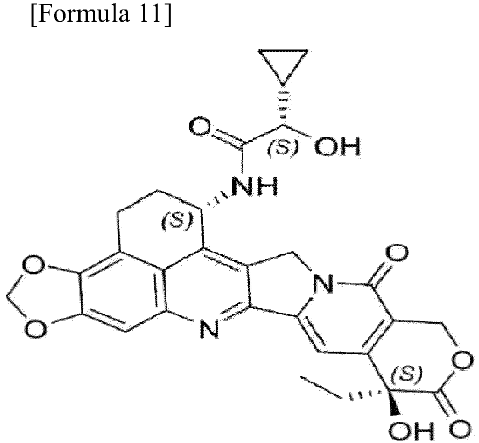

According to an aspect of the present invention, the compound of Formula 1 above may be an active camptothecin derivative designed to bind to DDX5 protein and E3 ligase. According to an aspect of the present invention, the compound of Formula 1 above may be one which binds to E3 ligase by a molecular glue.

According to an aspect of the present invention, the compound of Formula 1 above may kill the target cells expressing DDX5 protein through a molecular glue mechanism of action (MoA).

The target cells may be cancer cells or senescent cells. Senescent cells also include cells that no longer perform the organ's unique characteristic functions.

According to an aspect of the present invention, the compound of Formula 1 above may be one which has a mechanism of action (MoA) that degrades the oncoprotein DDX5 along with the ability to inhibit type 1 topoisomerase.

As used herein, the term "drug" or "payload" may refer to any one compound selected from the group consisting of Formulas 1 to 11. The drug or payload of the present invention may be combined with a linker and an anti-TF antibody to form an antibody-drug conjugate.

As used herein, the term "antibody-drug conjugate (ADC)" may enable the linkage of a monoclonal antibody or an antigen-binding fragment thereof with a biologically active cytotoxin via a chemically stable linker, and thereby avoid the low efficacy of antibody therapeutics or the toxicity issue of cytotoxin, while maximizing the specificity of antibody binding to surface antigens of cancer cells and the high efficiency of cytotoxin. This allows ADCs to accurately bind to cancer cells and reduce their impact on normal cells, compared to conventional anticancer treatments. The antibody-drug conjugate of the present invention specifically binds to cells expressing Tissue factor, and thus can specifically target drugs.

The antibody-drug conjugate is characterized in that it can easily penetrate cell membranes due to the hydrophobicity of the compound of Formula 1, and in that when it enters cancer cells, the linker is specifically cleaved by a lysosomal protease highly expressed in tumors, thereby releasing the drug of Formula 1 and killing the cancer cells.

The antibody-drug conjugate is characterized by having an excellent killing effect even on cancer cells with low Tissue factor (TF) expression, by causing a bystander effect, in which the drug is released after cell death and kills surrounding cancer cells.

The terms "tissue factor", "TF", "Tissue Factor", "CD142", "tissue factor antigen", "TF antigen" and "CD142 antigen" as used herein are used interchangeably herein and, unless otherwise specified, include any variants, isoforms and species homologs of human tissue factor that are naturally expressed by a cell or are expressed on a cell transfected with the tissue factor gene.

As used herein, the term "antibody" may refer to an antibody or an antigen-binding fragment thereof, or may refer to a macromolecule compound capable of recognizing and binding to an antigen or receptor associated with a population of target cells to which it binds. The antibody may be a murine antibody, a humanized antibody, a chimeric antibody, a rabbit antibody, a phage display antibody, a yeast display antibody, a CDR grafted antibody, or a recombinant human antibody, but is not limited thereto. In particular, the term antibody used herein refers to "anti-TF antibody", and this may refer to an antibody or an antigen-binding fragment thereof that specifically binds to Tissue factor (TF).

The antibody of the present invention may preferably be a monoclonal antibody, and the antigen-binding fragment may be one or more fragments of an antibody having specific binding ability to an antigen.

The antibody of the present invention may form a bond with a linker through a heteroatom on the antibody.

The antibody of the present invention is an anti-TF antibody, and non-limiting examples of the anti-TF antibody may be Tisotumab, or may be selected from the antibodies used in ADCs, such as XB-002 (Exelixis), MRG-004A (Shanghai Miracogen), AMB101 (AmMax), and STRO-004 (Sutro biopharma).

The antibody of the present invention may be Tisotumab.

The antibody of the present invention may be an antibody which includes i) a heavy chain including CDR-H1, CDR-H2, and CDR-H3 having the amino acid sequences of SEQ ID NOs: 1, 2, and 3, respectively, and ii) a light chain including CDR-L1, CDR-L2, and CDR-L3 having amino acid sequences of SEQ ID NOs: 4, 5, and 6, respectively; or an antigen-binding fragment thereof.

The antibody of the present invention may be an antibody which includes i) a heavy chain variable region having the amino acid sequence of SEQ ID NO: 7 and ii) a light chain variable region having the amino acid sequence of SEQ ID NO: 8; or an antigen-binding fragment thereof.

The antibody of the present invention may be an antibody which includes i) a heavy chain variable region having the amino acid sequence of SEQ ID NO: 9 and ii) a light chain variable region having the amino acid sequence of SEQ ID NO: 10; or an antigen-binding fragment thereof.

The drug to antibody ratio (DAR), which refers to the average number of drugs linked per antibody of the present invention, may be in a range of about 1 to about 20, and specifically, may be in a range of about 1 to about 8, about 2 to about 8, about 3 to about 8, about 4 to about 8, about 5 to about 8, about 6 to about 8, about 7 to about 8, or about 7.5 to about 8. Additionally, the DAR may specifically be in a range of about 3 to about 5, about 3.5 to about 4.5, or about 3 to about 4.

In an aspect of the present invention, the DAR of the antibody-drug conjugate of the present invention may be in a range of 7 to 8 or 3 to 4. In a preferred aspect of the present invention, the DAR of the antibody-drug conjugate of the present invention may be in a range of 7 to 8. In a preferred aspect of the present invention, the DAR of the antibody-drug conjugate of the present invention may be in a range of 3 to 4.

As used herein, the term "linker" refers to a chemical structural fragment or bond which is linked to an antibody at one end and to a drug at the other end, or linked to another linker and then to a drug.

More specifically, the linker may be cleavable or non-cleavable, and the cleavable linker not only functions to link the antibody and the drug together, but may also be selectively cleaved by at specific pH, enzymes (protease, phosphatase, glycosidase, and sulfatase), and a glutathione (GSH) level of the cancer-surrounding environment to thereby release the drug.

More specifically, the linker may include at least one selected from the group consisting of C₁₋₆ alkylene, carbonyl, maleimide, succinimide -O-, sugar, sugar acid, β-glucuronide, β-galactoside, phosphodiester, PEG, polysarcosine (PSAR), disulfide, *p-*aminobenzyloxycarbonyl (PAB), triazole, hydrazone, phosphate, diester, thioether, and peptide, but is not limited thereto.

More specifically, the peptide residue may be a peptide residue consisting of one or more amino acids selected from the group consisting of alanine (A), asparagine (N), glutamine (Q), ornithine, proline (P), threonine (T), phenylalanine (F), glycine (G), valine (V), lysine (K), citrulline, serine (S), glutamic acid (E), and aspartic acid (D); more specifically, it may be a peptide residue consisting of one or more amino acids selected from the group consisting of valine, citrulline, phenylalanine and glycine; more specifically, it may be a dipeptide, tripeptide, or tetrapeptide residue; and more specifically, it may be a dipeptide residue of valine-citrulline, valine-alanine, and phenylalanine-glycine, or a tetrapeptide residue of glycine-glycine-phenylalanine-glycine (GGFG). In this case, in the peptide residue, some of the amino acid residues may be optionally substituted.

The "linker" of the present invention may be represented by Formula 12 below: 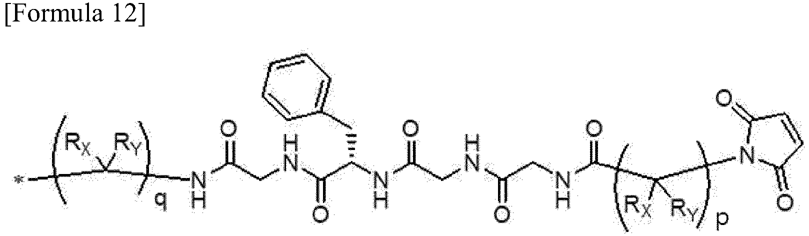 wherein in Formula 12 above,
R_{X} and R_{Y} are each independently hydrogen or C₁₋₆ alkyl;
p and q are each an integer of 1 to 6; and
* is a part that binds to the payload.

According to one aspect of the present invention, in Formula 12 above,
R_{X} and R_{Y} may each independently be hydrogen or C₁₋₃ alkyl;
p may be an integer of 4 to 6; and
q may be an integer of 1 to 3.
According to an aspect of the present invention, in Formula 12 above,
R_{X} and R_{Y} may each independently be hydrogen;
p may be 5; and
q may be 1.

According to an aspect of the present invention, the compound of Formula 12 above may be a compound of Formula 13.

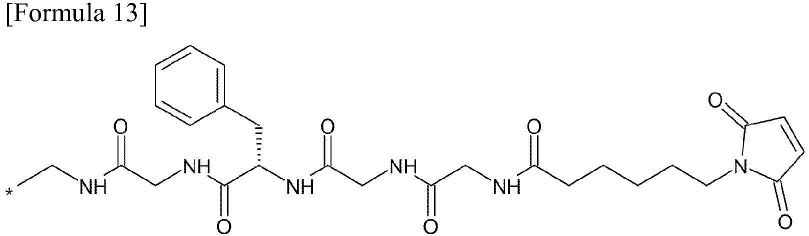

In Formula 13 above, * is a part that binds to the payload.

In an aspect of the present invention, in the compound of Formula 12, the maleimide ring may be linked to an amino acid residue of an antibody. In a preferred aspect of the present invention, in the compound of Formula 12, any one of the carbons of the CH₂=CH₂- structure of the maleimide ring may bind to cysteine among the amino acid residues of the antibody.

In an aspect of the present invention, when the compound of Formula 13 is combined with an antibody, it may be linked in the form of Formula 14.

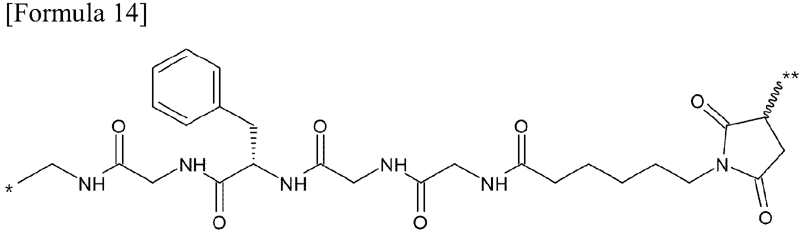

In Formula 14 above, * is a part that binds to the payload, and ** is a part that binds to the antibody.

In an aspect of the present invention, ** indicated in the compound of Formula 14 is a part that binds to an antibody. In a preferred aspect of the present invention, the compound of Formula 14 may bind to cysteine among the amino acid residues of the antibody at the ** part.

As used herein, the term "mc-GGFG" refers to a linker of the structure of a compound of Formula 13 or a compound of Formula 14.

The compound of Formula 1 according to the present invention may exist in the form of a pharmaceutically acceptable salt, stereoisomer, tautomer, prodrug, hydrate, solvate, or isotopically labeled analog. Accordingly, the scope of the compounds of the present invention may include the compound of Formula 1 above and a pharmaceutically acceptable salt, a stereoisomer, tautomer, prodrug, hydrate, solvate, or isotope-labeled analogs thereof, and may similarly be applied to drug-linker compounds and antibody-drug conjugates based thereon.

As used herein, the term "pharmaceutically acceptable salt" refers to any organic or inorganic acid addition salt of the compound, in a concentration that provides a relatively non-toxic and harmless to the patient and has an effective action, and the side effects due to the salt do not diminish the beneficial effects of the parent compound.

In particular, the pharmaceutically acceptable salt may be an acid addition salt formed by a free acid. In particular, the acid addition salt may be obtained from inorganic acids (*e.g*., hydrochloric acid, nitric acid, phosphoric acid, sulfuric acid, hydrobromic acid, hydroiodic acid, nitrous acid, phosphorous acid, *etc*.); non-toxic organic acids (*e.g*., aliphatic mono- and dicarboxylates, phenyl-substituted alkanoates, hydroxyalkanoates, and alkanedioates; aromatic acids; aliphatic and aromatic sulfonic acids, *etc*.); and organic acids (*e.g*., trifluoroacetic acid, acetate, benzoic acid, citric acid, lactic acid, maleic acid, gluconic acid, methanesulfonic acid, 4-toluenesulfonic acid, tartaric acid, fumaric acid, *etc*.).

Such pharmaceutically acceptable salts may include sulfates, sulfites, nitrates, phosphates, pyrophosphates, chlorides, bromides, iodides, fluorides, acetates, trifluoroacetates, propionates, decanoates, caprylates, acrylates, formates, isobutyrates, caprates, heptanoates, propiolates, oxalates, malonates, succinates, suberates, sebacates, fumarates, maleates, benzoates, phthalates, benzenesulfonates, toluenesulfonates, phenylacetates, phenylpropionates, phenylbutyrates, citrates, lactates, glycolates, malates, tartrates, mandelates, *etc*.

The acid addition salt may be prepared by a conventional method, for example, by dissolving the corresponding compound in an organic solvent (*e.g*., methanol, ethanol, acetone, methylene chloride, acetonitrile, *etc*.), adding an organic acid or inorganic acid thereto, and filtering and drying the resulting precipitate; or by distilling the solvent and an acid in an excess amount under reduced pressure, drying, and crystallizing in the presence of an organic solvent.

Additionally, the pharmaceutically acceptable salt may be a salt obtained using a base or a metal salt. As an example of the metal salt, an alkali metal or alkaline earth metal salt may be obtained by dissolving the compound in a solution with an excess amount of alkali metal hydroxide or alkaline earth metal hydroxide, filtering the undissolved compound salt, and evaporating and drying the filtrate. As the alkali metal salt, sodium, potassium, or calcium salts may be pharmaceutically suitable. Additionally, the corresponding salt may be obtained by reacting to an alkali metal or alkaline earth metal salt with a suitable silver salt (*e.g*., silver nitrate).

Additionally, the compound of the present invention may include hydrates and solvates. The hydrate refers to a compound or a salt thereof, which further includes a stoichiometric or non-stoichiometric amount of water bound by a non-covalent intermolecular force. The hydrate includes, for example, a monohydrate, a dihydrate, a trihydrate, a tetrahydrate, *etc*. The solvate refers to a solvent-containing compound formed by association of one or more solvent molecules with the compound of the present invention. The solvate includes, for example, a single solvate, a disolvate, a trisolvate, and a tetrasolvate. The hydrate and the solvate may be prepared using known methods, and are preferably non-toxic and water-soluble. In this case, preferably, the hydrate and solvate may each be water, an alcoholic solvent (especially, methanol, ethanol, *etc*.), trifluoroacetic acid, formic acid, and ammonia, but are not limited thereto.

Additionally, the compound of the present invention may be prepared in the form of a prodrug, which has little or no pharmacological activity itself, but can be converted into a compound having the desired activity, for example, by hydrolysis, when administered into the body. Such prodrugs may be derivatives of compounds including a biologically reactive functional group, in which the biologically reactive functional group may be cleaved from the compound or otherwise reacted under biological conditions (*in vivo* or *in vitro*) to provide the compound. Typically, prodrugs are inactive, or at least less active than the compound, which allows the compound to exhibit its activity after cleavage from the biologically reactive functional group. The biologically reactive functional group may be hydrolyzed or oxidized under biological conditions to produce a compound. For example, prodrugs may include a biologically hydrolyzable group. Examples of the biologically hydrolyzable groups may include biologically hydrolyzable phosphates, biologically hydrolyzable esters, biologically hydrolyzable amides, biologically hydrolyzable carbonic esters, biologically hydrolyzable carbamates, and biologically hydrolyzable ureides, but are not limited thereto. Additionally, the compound of the present invention may have the form of a stereoisomer. The stereoisomer refers to compounds which have the same formula or molecular formula but are sterically different, and include enantiomers, diastereomers, geometric isomers (or *cis*/*trans* isomers), rotamers, and atropisomers, and each of these isomers and mixtures thereof are also included in the scope of the present invention. For example, the compound of the present invention may have a chiral carbon center, and thus individual enanatiomers or mixtures thereof (*e.g*., racemic mixtures) may also fall within the scope of the present invention. Additionally, the compound of the present invention may have the form of a conformational isomer (*e.g*., a rotamer) and a diastereomer including a geometric isomer (or *cis*/*trans* isomer), and all such stereoisomers and mixtures thereof may all fall within the scope of the present invention. In this aspect, the Formula (I) of the present invention may include the stereoisomers of Formula (I) because the stereochemical structure is not specified. Specifically, the solid bond ( 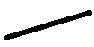 ) being linked to an asymmetric carbon atom in the chemical structural formula may include a wedge-shaped solid bond ( 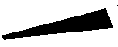 ) and a wedge-shaped dotted bond ( 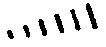 ), which represent the absolute arrangement of the stereogenic center. Additionally, it should be understood that stereoisomers of the exemplary compounds described herein are included within the scope of the present invention.

Additionally, the compound of the present invention may exist in the form of a tautomer. Tautomers may have structures in which a proton has shifted from one atom of a molecule to another atom of the same molecule, and may be interconverted by the movement of hydrogen atoms accompanying the conversion of single bonds and adjacent double bonds. In a solution where tautomerization is possible, a chemical equilibrium of tautomers will exist. The exact ratio of tautomers depends on several factors, including temperature, solvents, and pH.

Additionally, the compound of the present invention further includes an isotopically labeled analog. Specifically, the compound of the present invention may include isotopes of hydrogen, carbon, nitrogen, oxygen, phosphorus, sulfur, fluorine, and chlorine (²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively). All compounds of the present invention including the above-described isotopes and/or other isotopes of other atoms fall within the scope of the present invention.

According to an aspect of the present invention, there is provided a method for treating or preventing cancer, which includes contacting the antibody-drug conjugate with cancer cells.

The antibody-drug conjugate according to the present invention can inhibit the activity of cancer cells. Therefore, the present invention provides a pharmaceutical composition or anticancer agent for treating or preventing cancer, which includes the antibody-drug conjugate as an active ingredient.

Herein, "treatment" refers to any action whereby the symptoms of the disease are improved or beneficially changed by administration of the compound, and "prevention" refers to any action whereby the occurrence, spread, and recurrence of the disease are inhibited or delayed by administration of the compound.

Herein, "administration" refers to provision of a given substance to a subject in need thereof by any appropriate method, and the administration route of the antibody-drug conjugate of the present invention may be administered via any general route (*e.g*., oral or parenteral routes) as long as it can reach the target tissue.

Herein, "cancer" includes any cancer that can be treated by the inhibition of topoisomerase I, and/or inhibition of any one or more cancer-associated survival genes selected from the group consisting of DDX5, survivin, Mcl-1, XIAP, and cIAP2, and may be a solid cancer or hematological cancer. For example, the cancer may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, fallopian tube cancer, ovarian epithelial cancer, ovarian germ cell cancer, male breast cancer, brain cancer, pituitary adenoma, multiple myeloma, gallbladder cancer, biliary tract cancer, colorectal cancer, head and neck cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, paranasal sinus and nasal cavity cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, uretheral cancer, cancer of unknown primary site, gastric lymphoma, gastric cancer, gastric carcinoid, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi's sarcoma, Paget's disease, tonsil cancer, squamous cell carcinoma, pulmonary adenocarcinoma, lung cancer, pulmonary squamous cell carcinoma, skin cancer, anal cancer, rhabdomyosarcoma, laryngeal cancer, pleural cancer, blood cancer, and thymic cancer, but is not limited thereto. Additionally, the cancer includes not only primary cancer but also metastatic cancer.

According to an aspect of the present invention, the cancer may be one or more selected from the group consisting of cervical cancer, endometrial cancer, ovarian cancer, peritoneal cancer, fallopian tube cancer, breast cancer, head and neck cancer, esophageal cancer, bladder cancer, and pancreatic cancer

In another aspect, the present invention relates to a method for treating or preventing cancer, which includes administering the antibody-drug conjugate to a subject.

As used herein, the term "subject" is used synonymously with "individual", and the subject may be a mammal, for example, a human, a mouse, a cow, a horse, a pig, a dog, a sheep, a goat, or a cat.

Any description that overlaps with the pharmaceutical composition of the present invention described above is omitted.

In still another aspect, the present invention relates to the use of the antibody-drug conjugate for treating or preventing cancer.

Any description that overlaps with the pharmaceutical composition of the present invention described above is omitted.

In still another aspect, the present invention relates to the use of the antibody-drug conjugate in preparation of a pharmaceutical composition for treating or preventing cancer.

Any description that overlaps with the pharmaceutical composition of the present invention described above is omitted.

All matters mentioned in the compositions, treatment methods, and uses of the present invention apply equally unless they are contradictory to one another.

### Mode for Carrying Out the Invention

### [Examples]

Hereinafter, the constitution of the present invention is specifically described through examples; however, the following examples are only intended to help understanding of the present invention and the scope of the present invention is not limited to these examples. The reagents, solvents, and starting materials described in the examples below, if they are substances that can be easily purchased, their sources of acquisition are not specifically described.

The method for preparing a camptothecin derivative according to the present invention is as follows.

### Preparation Examples: Synthesis of PBX Payload

### Preparation Example 1: Synthesis of Active Camptothecin Derivatives of Formula 2 or Formula 3 (PBX-7011 and PBX-7012)

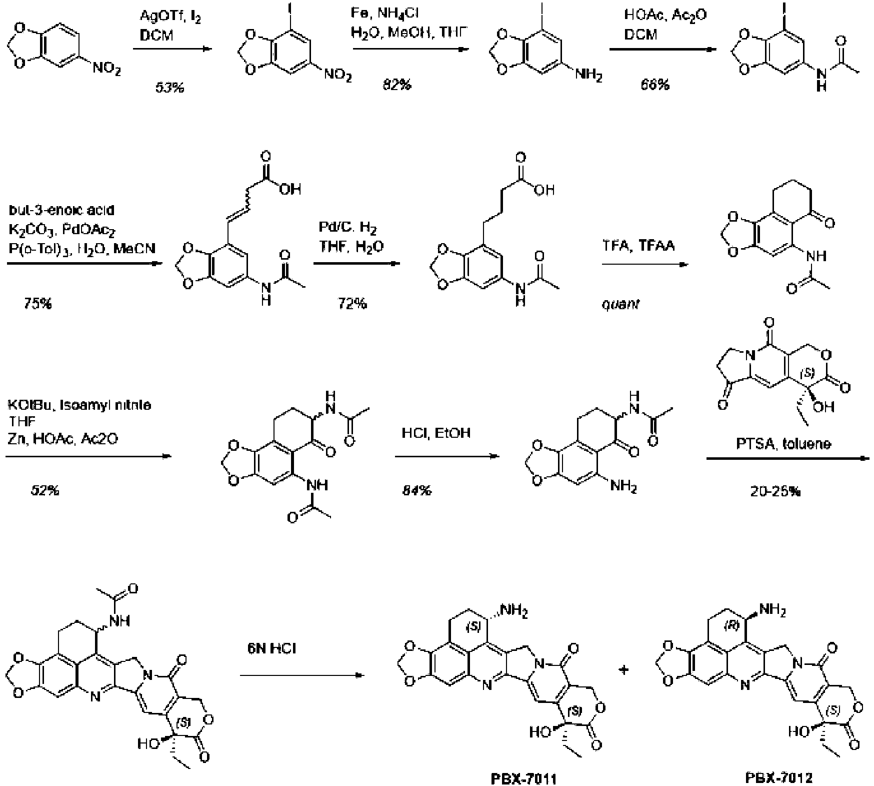

### 1-1. Synthesis of Compound 2

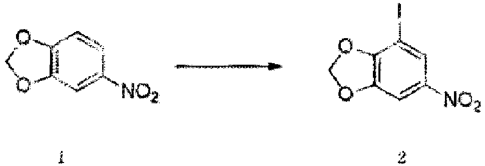

Silver triflate (57.7 g, 224 mmol) and iodine (57.0 g, 224 mmol) were added to a solution of 5-nitrobenzo[d][1,3]dioxole (25 g, 150 mmol) in a dichloromethane (748 mL) flask. The solution was stirred in the dark at room temperature under a N₂ atmosphere.

AgI was removed by filtration and the solid was washed with dichloromethane (100 mL). The solvent was removed under reduced pressure and the residue was partitioned between EtOAc (250 mL) and 5% (v/v) a NH₄OH/H₂O solution (200 mL). The organic layer was separated, washed with 1 M Na₂SO₃ (5 × 200 mL) and brine (200 mL), dried over Na₂SO₄, treated with activated charcoal, and filtered through Celite. The solvent was evaporated under reduced pressure to obtain the crude product as a brown solid. This was triturated in ice-cold EtOH (400 mL), filtered, and the solid was washed with ice-cold EtOH (100 mL) to give the product as a pale brown-gray solid (14.3 g). The solvent was removed from the residue and the crude product was triturated a second time in ice-cold EtOH (300 mL). The solid was recovered by filtration and washed with EtOH (50 mL) to obtain an additional amount of the product (10.2 g) as a dark brown-gray solid. Both batches were used as-is without further purification.

SC_ACID: m/z 294.2 [M+H]⁺

### 1-2. Synthesis of Compound 3

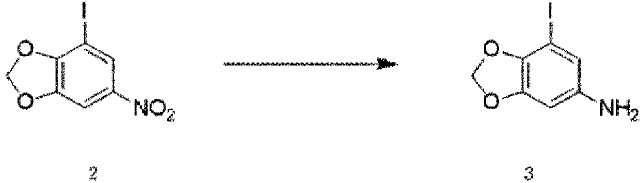

To a suspension of 4-iodo-6-nitrobenzo[d][1,3]dioxole (8.75 g, 29.9 mmol) in a mixture of water (80 mL), methanol (40.0 mL), and tetrahydrofuran (40.0 mL), iron powder (6.67 g, 119 mmol) and ammonium chloride (6.39 g, 119 mmol) were added. The suspension was heated to 75°C and stirred for 16.5 hours. The reaction mixture was cooled to room temperature and concentrated under reduced pressure. The resulting black solid was suspended in ethyl acetate (100 mL) and the solution was decanted. The residue was washed with EtOAc (3 × 50 mL). The mixture was transferred to a separatory funnel, and water was added thereto, and the aqueous layer was removed. The organic layer was washed with saturated aqueous NaHCO₃ solution (150 mL) and brine (150 mL). The organic layer was dried over Na₂SO₄ and the solvent was removed under reduced pressure to obtain a brown solid (4.75 g, 60% yield). An additional product was recovered by thoroughly washing the iron residue with ethyl acetate. The organic fraction was then washed with a saturated aqueous NaHCO₃ solution (150 mL), brine (150 mL), and dried over Na₂SO₄. The solvent was removed under reduced pressure to obtain a brown solid (1.74 g, 22% yield).

SC_ACID: m/z 264.0 [M+H]⁺

### 1-3. Synthesis of Compound 4

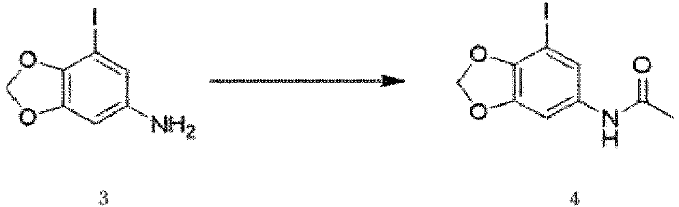

To a solution of 7-iodobenzo[d][1,3]dioxol-5-amine (6.39 g, 24.29 mmol) in dichloromethane (49 mL), acetic anhydride (2.75 mL, 29.2 mmol) and triethylamine (4.06 mL, 29.2 mmol) were added. The reaction mixture was stirred at room temperature overnight. After several hours, additional DCM (10 mL) was added thereto. The suspension was filtered through a sintering funnel and washed with ice-cold DCM (10 mL). The product was further dried under reduced pressure to obtain a light gray solid (4.46 g). The filtrate was concentrated under reduced pressure, and the residue was dissolved in EtOAc, washed with water and brine, dried over Na₂SO₄, and concentrated under reduced pressure to obtain a brown solid (~3 g). The brown solid was purified by flash column chromatography (80 g Si, 0-100% ethyl acetate in heptane). All batches of the product were triturated in ice-cold EtOAc (5-10 mL). The two batches were combined and further dried to obtain an off-white solid.
Total yield: 5.0 g, 66%
SC_ACID: m/z 306.0 [M+H]⁺
¹H NMR (400 MHz, DMSO-d6) δ 9.88 (s, 1H), 7.39 (d, J = 1.9 Hz, 1H), 7.17 (d, J = 1.9 Hz, 1H), 6.04 (s, 2H), 1.99 (s, 3H).

### 1-4. Synthesis of Compound 5

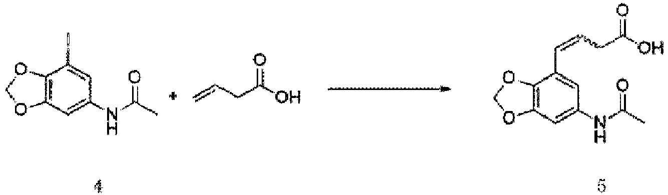

A slurry of N-(7-iodobenzo[d][1,3]dioxol-5-yl)acetamide (5.06 g, 16.59 mmol), *but*-3-enoic acid (1.69 mL, 19.90 mmol), and potassium carbonate (2.98 g, 21.56 mmol) in acetonitrile (40 mL) was cooled to 0°C to 5°C in a 3-neck flask equipped with a condenser. Water (13.33 mL) was slowly added thereto to generate gas. When gas generation stopped, the mixture was degassed with Ar for 30 minutes. Tri-*o-*tolylphosphine (0.505 g, 1.659 mmol) and palladium acetate (0.186 g, 0.829 mmol) were added and the mixture was degassed for another 30 minutes and then heated to reflux under Ar. The reaction mixture was cooled to room temperature and filtered through Celite. The filter cake was washed with H₂O and EtOAc. The organic solvent was removed from the filtrate under vacuum and the aqueous solution was acidified to a concentrated solution. The aqueous layer of HCl was extracted with EtOAc until the pH became 1 to 2, and the combined organic phases were washed with brine, dried over Na₂SO₄, filtered and concentrated under reduced pressure to obtain the crude product. The crude material was triturated in ice-cold EtOAc (30 mL) and the solid was recovered by filtration to obtain the product as a brown solid. The mother liquor was concentrated under reduced pressure and purified by flash chromatography (80 g Si, 0-100% EtOAc in heptane). The product fractions were concentrated under reduced pressure to obtain a light brown foam. A mixture of E/Z isomers was obtained (total yield: 3.46 g, 75%).

SC_ACID: m/z 264.4 [M+H]⁺

### 1-5. Synthesis of Compound 6

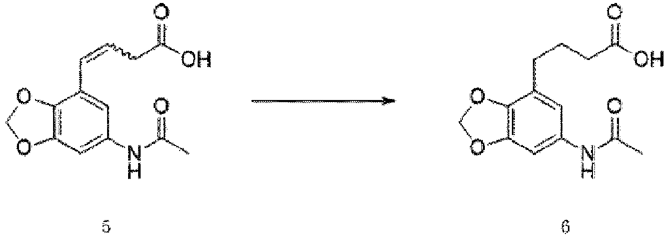

A suspension of 4-(6-acetamidobenzo[d][1,3]dioxol-4-yl)*but*-3-enoic acid (3.47 g, 13.18 mmol) in tetrahydrofuran (50 mL)/water (50 mL) was degassed with N₂ for 15 minutes. Pd/C (2.97 g, 1.397 mmol) was added before heating to 40°C under H₂ (balloon) and the suspension was degassed with H₂ for 5 minutes. After 24 hours, nitrogen and additional Pd/C (2.97 g, 1.397 mmol) were added to the reaction mixture. The reaction mixture was bubbled with hydrogen for 10 minutes and stirred overnight at 45°C under hydrogen. The reaction mixture was filtered through Celite. The filter cake was washed with H₂O (50 mL). EtOAc (50 mL) and the organic solvent was removed from the filtrate under vacuum. The aqueous solution was acidified with concentrated HCl until pH became 1 and the dark brown/green precipitate was collected by filtration through a sintering funnel. The aqueous filtrate was extracted. The combined organic phases were washed with brine, dried over Na₂SO₄, filtered, and the solvent was removed under vacuum to obtain brown oil. Since the product recovery rate was low, the filter cake was washed with EtOAc (50 mL), water (200 mL), and MeOH (400 mL). The water/EtOAc flush was added to the flask containing the solid from the MeOH flush. The aqueous layer was acidified to a high concentration. An off-white precipitate continued to form until the pH was adjusted to 1 using HCl, and it was collected by filtration through a sintering funnel. After extracting the aqueous filtrate with EtOAc (3 × 200 mL), LCMS showed that all products were removed in the aqueous phase. The combined organic phases were washed with brine, dried over Na₂SO₄, and filtered, and the solvent was removed under vacuum to obtain a light brown solid. All product batches were combined and purified by flash column chromatography (40 g Si, 0-10% MeOH in DCM). The product fractions were concentrated to obtain a light brown solid (Yield: 2.52 g, 72%).
SC_ACID: 266.2 [M+H]⁺
¹H NMR (400 MHz, DMSO-d6) δ 12.07 (s, 1H), 9.78 (s, 1H), 7.14 (d, J = 2.0 Hz, 1H), 6.80 (d, J = 2.2 Hz, 1H), 5.95 (s, 2H), 2.50 - 2.46 (m, 2H), 2.23 (t, J = 7.4 Hz, 2H), 1.98 (s, 3H), 1.84 - 1.70 (m, 2H).

### 1-6. Synthesis of Compound 7

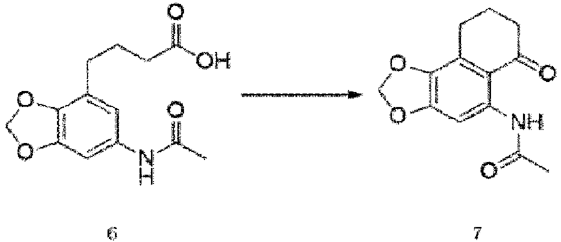

A suspension of 4-(6-acetamidobenzo[d][1,3]dioxol-4-yl)butanoic acid (150 mg, 0.565 mmol) in TFA (433 µL, 5.65 mmol) was cooled on ice. TFAA (157 µL, 1.131 mmol) was added thereto. The mixture was stirred at 0°C to 5°C for 1 hour, and it turned into a dark solution over time. The reaction mixture was added dropwise to an ice-cold saturated aqueous NaHCO₃ solution (10 mL), and the aqueous solution was extracted with ethyl acetate (3 × 25 mL). The saturated NaHCO₃ of combined organic layers and brine were dried over Na₂SO₄, filtered, and the solvent was removed under vacuum to obtain a salmon pink solid (Yield: 140 mg, 100%).
SC_ACID: m/z 248.2 [M+H]⁺
¹H NMR (400 MHz, DMSO-d6) δ 12.34 (s, 1H), 8.11 (s, 1H), 6.13 (s,2H), 2.80 (t, J = 6.2 Hz, 2H), 2.66 - 2.58 (m, 2H), 2.12 (s, 3H), 2.01 - 1.91(m, 2H).

### 1-7. Synthesis of Compound 8

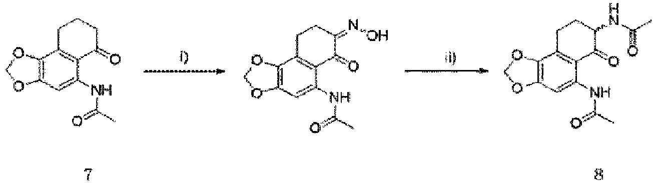

A suspension of N-(6-oxo-6,7,8,9-tetrahydronaphtho[1,2-d][1,3]dioxol-5-yl)acetamide (50 mg, 0.202 mmol) in tetrahydrofuran (1.2 mL) was cooled to 0°C and potassium tert-butoxide (27.2 mg, 0.243 mmol) and isoamyl nitrite (35.0 µL, 0.263 mmol) were added thereto. The dark green mixture was stirred on ice (< 5°C) for 1.5 hours. Acetic acid (170 µL, 2.94 mmol), acetic anhydride (170 µL, 1.810 mmol), and zinc dust (66.1 mg, 1.011 mmol) were added to the reaction mixture. The suspension was stirred at 0°C for 2 hours. The reaction mixture was filtered over Celite and flushed with DCM. The filtrate was concentrated under reduced pressure to obtain a black oily substance. The crude product was purified by flash column chromatography (4 g Si, 0-4% MeOH in DCM). The product fractions were concentrated to obtain a gray solid (32 mg, 52%) with 80-90% purity. Higher purity samples can be obtained by preparative MPLC.
SC_ACID: m/z 305.4 [M+H]⁺
¹H NMR (400 MHz, DMSO-d6) δ 12.10 (s, 1H), 8.21 (d, J = 8.0Hz, 1H), 8.12 (s, 1H), 6.15 (d, J = 9.3 Hz, 2H), 4.66 - 4.56 (m, 1H), 2.93(dd, J = 8.9, 4.0 Hz, 2H), 2.14 (s, 3H), 2.13 - 1.93 (m, 2H), 1.91 (s, 3H).

### 1-8. Synthesis of Compound 9

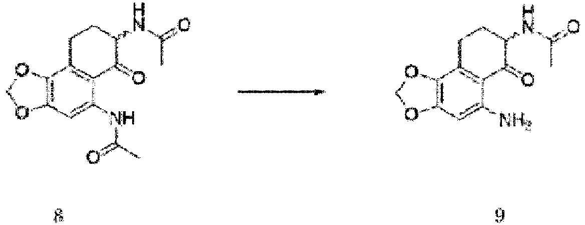

N,N'-(6-Oxo-6,7,8,9-tetrahydronaphtho[1,2-d][1,3]dioxole-5,7-diyl)diacetamide (244 mg, 0.802 mmol) was suspended in 2 M hydrochloric acid (4.69 mL, 9.38 mmol) in ethanol/water (5/1). The mixture was heated to 55°C for 4 hours. The black mixture was cooled to 0°C to 5°C. Triethylamine (1.4 mL, 10.04 mmol) was added dropwise while stirring. The mixture was then diluted with EtOH and evaporated to dryness. The residue was partitioned between water and DCM. The layers were separated and the aqueous layer was extracted once with DCM. The combined organic layers were dried over Na₂SO₄ and concentrated to obtain the product as a brown solid (178 mg, 73%) in 86% purity.
SC_ACID: m/z 263.0 [M+H]⁺
¹H NMR (400 MHz, DMSO) δ 8.04 (d, J = 8.0 Hz, 1H), 6.20 (s, 1H), 5.94 (d, J = 6.0 Hz, 2H), 4.48 - 4.41 (m, 1H), 3.08 (s, 2H), 2.88 - 2.69(m, 2H), 2.15 - 2.03 (m, 1H), 1.88 (s, 3H), 1.86 - 1.75 (m, 1H).

### 1-9. Synthesis of Compound 10

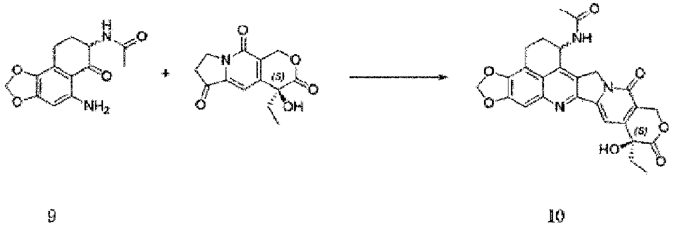

(4S)-4-Ethyl-7,8-dihydro-4-hydroxy-1H-pyrano[3,4-f]indolizin-3,6,10(4H)-trione (146 mg, 0.555 mmol) and N-(5-amino-6-oxo-6,7,8,9-tetrahydronaphtho[1,2-d][1,3]dioxol-7-yl)acetamide (112 mg, 0.427 mmol) were added to dry toluene (4.5 mL). PPTS (21 mg, 0.085 mmol) was added thereto and the reaction mixture was stirred at 115°C for 40 hours.

The reaction mixture was cooled to room temperature. The suspension was diluted with 2 mL DCM and filtered. A black residue (210 mg) was obtained.

The crude product was purified by column chromatography (12 g Si, 0-7% methanol in DCM) to obtain the product (45 mg, 21%) as a brown solid.

LCMS analysis showed two diastereoisomers.
SC_ACID: m/z 490.2 [M+H]⁺
¹H NMR (400 MHz, DMSO-d6) δ 8.47 (t, J = 9.3 Hz, 1H), 7.42 (s, 1H), 7.24 (s, 1H), 6.49 (s, 1H), 6.29 (d, J = 5.2 Hz, 2H), 5.57 - 5.49 (m, 1H), 5.41 (s, 2H), 5.23 - 5.07 (m, 2H), 3.09 - 3.00 (m, 2H), 2.11 - 2.01 (m, 2H), 1.91 (s, 3H), 1.89 - 1.79 (m, 2H), 0.87 (t, J = 7.1 Hz, 3H).

### 1-10. Synthesis of PBX-7011 and PBX-7012

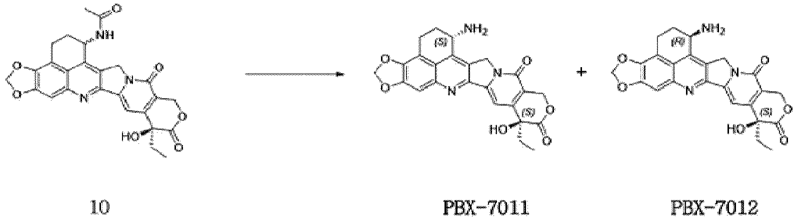

N-((10S)-10-ethyl-10-hydroxy-11,14-dioxo-2,3,10,11,14,16-hexahydro-1H,13H-benzo[de][1,3]dioxolo[4,5-g]pyrano[3',4':6,7]indolizino[1,2-b]quinolin-1-yl)acetamide (73.5 mg, 0.150 mmol) was dissolved in 1.0 mL 6 N HCl and stirred at 90°C for 8 hours and then at room temperature overnight. The reaction mixture was concentrated under reduced pressure and purified by acidic preparative MPLC (Luna2-30) run twice. The fractions including the separated diastereomers were acidified with 5 drops of 3 N HCl and lyophilized to obtain the product as a yellow solid.
1st eluting isomer: PBX-7011, 23 mg, 34% yield
   U_AN_ACID: m/z 448.4 [M+H]⁺
   ¹H NMR (400 MHz, DMSO-d6) δ 8.54 (s, 3H), 7.50 (s, 1H), 7.27 (s, 1H), 6.52 (s, 1H), 6.34 (d, J = 13.3 Hz, 2H), 5.77 (d, J = 19.3 Hz, 1H), 5.44(s, 2H), 5.37 (d, J = 19.3 Hz, 1H), 5.05 (s, 1H), 3.19 - 3.02 (m, 2H), 2.46(s, 1H), 2.20 - 2.03 (m, 1H), 1.94 - 1.81 (m, 2H), 0.88 (t, J = 7.3 Hz, 3H).
2nd eluting isomer: PBX-7012, 28 mg, 41% yield
   U_AN_ACID: m/z 448.2 [M+H]^{+ 1}H NMR (400 MHz, DMSO-d6) δ 8.57 (d, J = 4.7 Hz, 3H), 7.51 (s, 1H), 7.27 (s, 1H), 6.52 (s, 1H), 6.34 (d, J = 12.2 Hz, 2H), 5.76 (d, J = 19.4 Hz, 1H), 5.44 (s, 2H), 5.37 (d, J = 19.4 Hz, 1H), 5.08 (s, 1H), 3.16 - 2.98(m, 2H), 2.46 (s, 1H), 2.18 - 2.06 (m, 1H), 1.94 - 1.80 (m, 2H), 0.87 (t, J = 7.3 Hz, 3H).

### Preparation Example 2: Synthesis of Active Camptothecin Derivatives of Formula 4 or Formula 5 (PBX-7014 and PBX-7015)

This Example describes the synthesis of compounds PBX-7014 and PBX-7015 starting from two separated diastereomers of the Exatecan-hybrid compounds (PBX-7011 and PBX-7012).

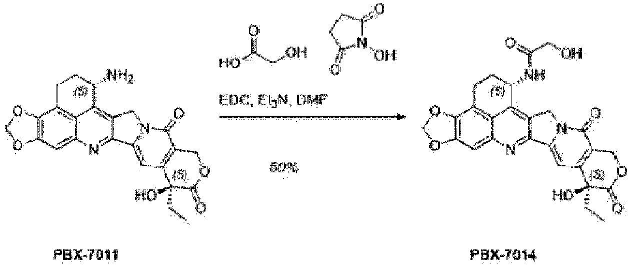

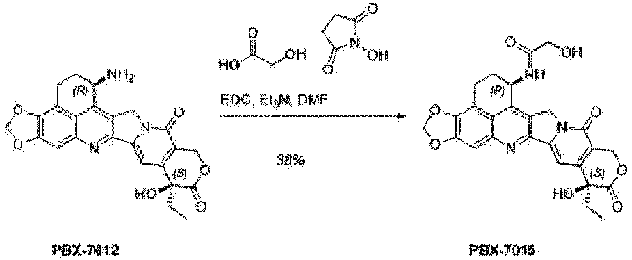

### 2-1: Synthesis of PBX-7014

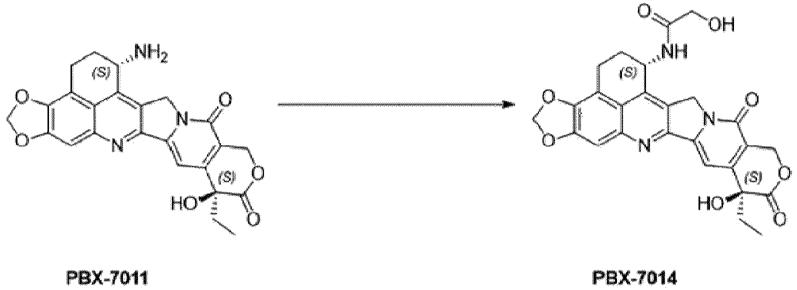

A stock solution of an activated glycolic acid was prepared according to the following procedure:
Glycolic acid (17 mg, 0.224 mmol) was dissolved in 1 mL of N,N-dimethylformamide. HOSu (25.7 mg, 0.223 mmol) and EDC (42.8 mg, 0.223 mmol) were added thereto. The reaction mixture was stirred at room temperature for 1 hour.

Next, 0.4 mL of the activated acid solution was added to a suspension of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (40 mg, 0.089 mmol) and triethylamine (0.025 mL, 0.179 mmol) in N,N-dimethylformamide (2.5 mL). The mixture was stirred at room temperature for 3 hours. 0.05 mL of a freshly prepared activated acid solution was added thereto. Then, the reaction mixture was stirred at room temperature for an additional 2 hours. The reaction mixture was evaporated to dryness. The crude product was purified by column chromatography (0-8% methanol in chloroform). This gave a yellow solid which includes the PBX-7014 product and residual succinimide. The product was further purified by acid preparative MPLC (Luna5-40) and the product fractions were lyophilized to obtain a light yellow solid (Yield: 20 mg, 50%).
U_AN_ACID: m/z 506.2 [M+H]⁺
¹H NMR (400 MHz, DMSO-d6) δ 8.40 (d, J = 8.9 Hz, 1H), 7.40 (s, 1H), 7.23 (s, 1H), 6.49 (s, 1H), 6.28 (d, J = 4.6 Hz, 2H), 5.61 - 5.45 (m, 2H), 5.45 - 5.35 (m, 2H), 5.19 - 5.06 (m, 2H), 3.95 (s, 2H), 3.13 - 2.96 (m, 2H), 2.21 - 2.02 (m, 2H), 1.94 - 1.77 (m, 2H), 0.87 (t, J = 7.3 Hz, 3H).

### 2-2: Synthesis of PBX-7015

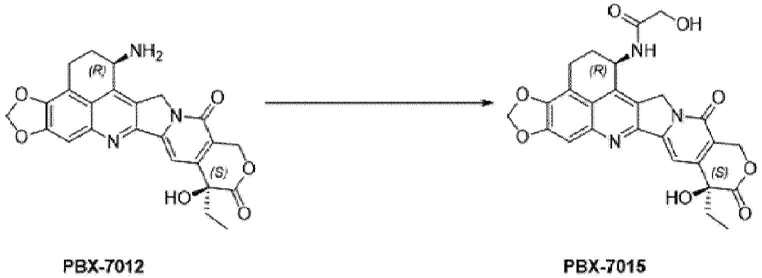

A stock solution of an activated acid was prepared according to the following procedure:
Glycolic acid (17.00 mg, 0.223 mmol) was dissolved in 1 mL of N,N-dimethylformamide. HOSu (25.7 mg, 0.223 mmol) and EDC (42.8 mg, 0.223 mmol) were added thereto. The reaction mixture was stirred at room temperature for 1 hour.

Next, 0.25 mL of the activated acid solution was added to a suspension of (1R,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (25 mg, 0.056 mmol) and triethylamine (0.016 mL, 0.112 mmol) in N,N-dimethylformamide (2.5 mL). The mixture was stirred at room temperature overnight. 0.03 mL of a freshly prepared activated acid solution was added thereto. The reaction mixture was then stirred at room temperature for 2 hours. The reaction mixture was combined with the previous smaller batch and evaporated to dryness. The crude product was purified by column chromatography. This gave a yellow solid including the PBX-7015 product and residual succinimide. The product was purified by acid preparative MPLC (Luna5-40). The product fractions were lyophilized to obtain a light yellow solid (Yield: 15 mg, 38%).
U_AN_ACID: 506.2 [M+H]⁺
¹H NMR (400 MHz, DMSO) δ 8.44 (d, J = 9.0 Hz, 1H), 7.41 (s, 1H), 7.24 (s, 1H), 6.48 (s, 1H), 6.29 (d, J = 2.3 Hz, 2H), 5.61 - 5.44 (m, 2H), 5.44 - 5.36 (m, 2H), 5.20 - 5.07 (m, 2H), 3.96 (s, 2H), 3.10 - 2.96 (m, 2H), 2.20 - 2.06 (m, 2H), 1.95 - 1.79 (m, J = 7.3 Hz, 2H), 0.87 (t, J = 7.3 Hz, 3H).

### Preparation Example 3: Synthesis of Active Camptothecin Derivative of Formula 6 (PBX-7016) or Synthesis of Active Camptothecin Derivative of Formula 7 (PBX-7017)

This Example describes the synthesis of Compound PBX-7016 starting from an Exatecan-hybrid compound (PBX-7011).

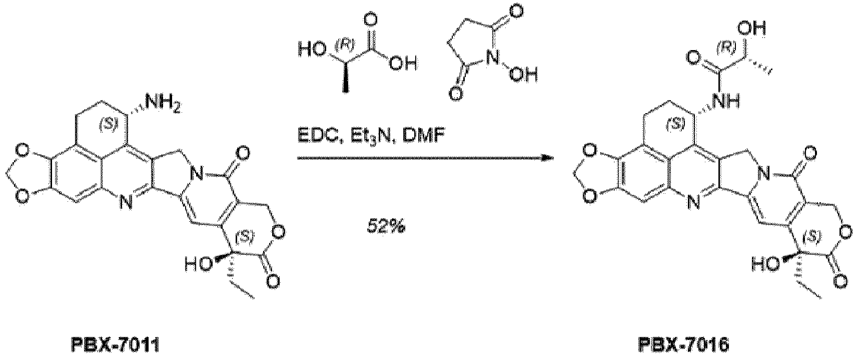

A stock solution of the activated D-lactic acid was prepared according to the following procedure.

D-Lactic acid (22 mg, 0.244 mmol) was dissolved in 1 mL of N,N-dimethylformamide. HOSu (27 mg, 0.235 mmol) and EDC (38.6 mg, 0.201 mmol) were added thereto. The reaction mixture was stirred at room temperature for 2 hours.

Next, 0.3 mL of the activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (36 mg, 0.080 mmol) and triethylamine (0.022 mL, 0.161 mmol) in N,N-dimethylformamide (2.5 mL). The mixture was stirred at room temperature for 6 hours. 0.05 mL of the prepared activated acid solution was added thereto. Then, the reaction mixture was stirred at room temperature overnight. The reaction mixture was purified directly by acid preparative MPLC (Luna10-50), and the product fractions were lyophilized to obtain a light yellow solid.
Yield: 22mg, 52%
U_AN_ACID: m/z 520.2 [M+H]⁺
¹H NMR (400 MHz, DMSO) δ 8.43 (d, J = 9.1 Hz, 1H), 7.41 (s, 1H), 7.23 (s, 1H), 6.50 (s, 1H), 6.29 (d, J = 2.1 Hz, 2H), 5.62 - 5.58 (m, 1H), 5.58 - 5.51 (m, 1H), 5.41 (s, 2H), 5.21 - 5.01 (m, 2H), 4.17 - 4.07 (m, 1H), 3.14 - 2.95 (m, 2H), 2.19 - 2.04 (m, 2H), 1.92 - 1.78 (m, 2H), 1.39 (d, J = 6.8 Hz, 3H), 0.87 (t, J = 7.3 Hz, 3H).

As described above, since PBX-7016 of Formula 6 can be synthesized from PBX-7011 of Formula 2, PBX-7017 of Formula 7 can be synthesized from PBX-7012 of Formula 3 in the same manner.

### Preparation Example 4: Synthesis of Active Camptothecin Derivative of Formula 8 (PBX-7018)

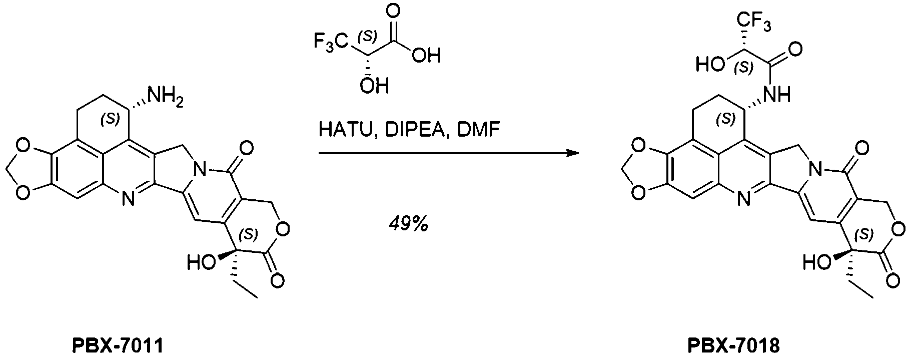

(S)-3,3,3-Trifluoro-2-hydroxypropanoic acid (11.59 mg, 0.080 mmol) was dissolved in N,N-dimethylformamide (2 mL) and hexafluorophosphate azabenzotriazole tetramethyl uronium (HATU) (30.6 mg, 0.080 mmol) was added thereto.

After the mixture was stirred for 3 minutes, (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (which was named PBX-7011) (30 mg, 0.067 mmol) and N,N-Diisopropylethylamine (DIPEA) (0.047 mL, 0.268 mmol) were added thereto, and the reaction mixture was stirred at room temperature for 1.5 hours. The reaction mixture was purified directly by acid preparative MPLC (Luna10-50) and the product fractions were lyophilized to obtain the product as an off-white solid (Yield: 19 mg, 49%).

The PBX-7011 can be obtained by the method described in PCT/KR2023/009854, *etc*., but the method is not limited thereto.
U_AN_ACID: m/z 574.2 [M+H]⁺
¹H NMR (400 MHz, DMSO-d6) δ 8.95 (d, *J* = 8.6 Hz, 1H), 7.42 (s, 1H), 7.24 (s, 1H), 7.18 (d, *J* = 6.2 Hz, 1H), 6.48 (s, 1H), 6.29 (d, *J* = 7.1 Hz, 2H), 5.61 - 5.52 (m, 1H), 5.46 - 5.35 (m, 2H), 5.21 (d, *J* = 19.2 Hz, 1H), 5.06 (d, *J* = 19.2 Hz, 1H), 4.66 - 4.55 (m, 1H), 3.08 - 2.97 (m, 2H), 2.18 - 2.03 (m, 2H), 1.93 - 1.77 (m, 2H), 0.87 (t, *J* = 7.3 Hz, 3H).

### Preparation Example 5: Synthesis of Active Camptothecin Derivative of Formula 9 (PBX-7020)

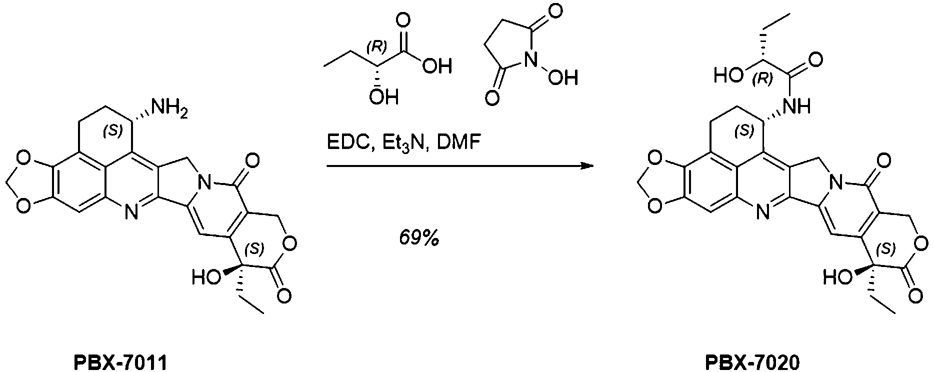

(R)-2-Hydroxybutyric acid (20.9 mg, 0.201 mmol) was dissolved in 1 mL DMF. HOSu (23.1 mg, 0.201 mmol) and EDC (38.6 mg, 0.201 mmol) were added, and the reaction mixture was stirred at room temperature for 2 hours.

Next, 0.3 mL of the activated acid solution was added to a suspension of PBX7011 (30 mg, 0. 067 mmol) and triethylamine (0.019 ml, 0.134 mmol) in N,N-dimethylformamide (2 mL). The mixture was stirred at room temperature for 23 hours. The reaction mixture was purified directly by acidic preparative MPLC (Luna10-50), which gave an off-white solid after lyophilization of the product fractions (Yield: 25 mg, 69%).
U_AN_ACID: m/z 534.2 [M+H]⁺
¹H NMR (400 MHz, DMSO) δ 8.39 (d, J = 8.9 Hz, 1H), 7.41 (s, 1H), 7.23 (s, 1H), 6.48 (s, 1H), 6.29 (d, J = 3.6 Hz, 2H), 5.57 - 5.50 (m, 1H), 5.48 - 5.44 (m, 1H), 5.41 (s, 2H), 5.13 (s, 2H), 3.92 - 3.86 (m, 1H), 3.06 - 3.00 (m, 2H), 2.15 - 2.05 (m, 2H), 1.92 - 1.74 (m, 3H), 1.71 - 1.62 (m, 1H), 0.93 (t, J = 7.4 Hz, 3H), 0.87 (t, J = 7.3 Hz, 3H).

### Preparation Example 6: Synthesis of Active Camptothecin Derivative of Formula 10 (PBX-7022)

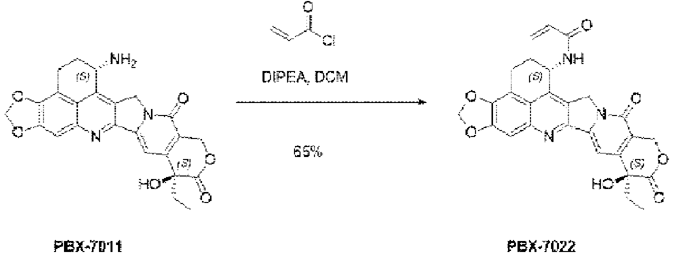

A suspension of PBX-7011 (30 mg, 0. 067 mmol) and DIPEA (0.047 mL, 0.268 mmol) in dichloromethane (dry) (1 mL) was cooled to 0°C. A solution of acryloyl chloride (5.44 µL, 0.067 mmol) was added dropwise to 0.1 mL of DCM. The reaction mixture was stirred at 0°C, and after 30 minutes, 0.15 equivalents of acrylonitrile chloride were added to stock solution of DCM. After stirring for a total of 2 hours, the reaction mixture was concentrated under reduced pressure. The residue was dissolved in DMSO and purified by acid preparative MPLC (Reprosil 10-50 method). The product fractions were collected and lyophilized to obtain a light yellow fluffy solid (Yield: 22 mg, 65%).
U_AN_ACID: m/z 502.2 [M+H]⁺
¹H NMR (400 MHz, DMSO-d6) δ 8.71 (d, *J* = 8.8 Hz, 1H), 7.43 (s, 1H), 7.23 (s, 1H), 6.49 (s, 1H), 6.30 (d, *J* = 4.2 Hz, 2H), 6.28 - 6.21 (m, 2H), 5.69 (dd, *J* = 8.5, 3.7 Hz, 1H), 5.65 - 5.58 (m, 1H), 5.40 (s, 2H), 5.25 - 5.12 (m, 1H), 5.12 - 4.99 (m, 1H), 3.10 - 3.02 (m, 2H), 2.16 - 2.06 (m, 2H), 1.92 - 1.78 (m, 2H), 0.86 (t, *J* = 7.3 Hz, 3H).

### Preparation Example 7: Synthesis of Active Camptothecin Derivative of Formula 11 (PBX-7024)

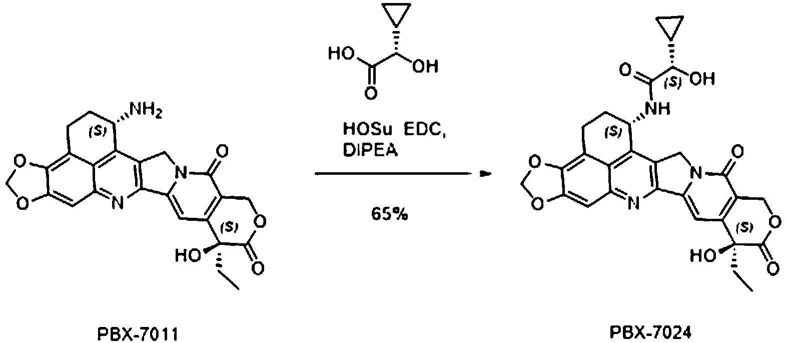

PBX-7024 was prepared in high yield by linking (2S)-2-cyclopropyl-2-hydroxyacetic acid to the PBX-7011 compound.

(2S)-2-Cyclopropyl-2-hydroxyacetic acid (26 mg, 0.244 mmol) was dissolved in 1 mL of N,N-dimethylformamide. HOSu (26 mg, 0.226 mmol) and EDC (42 mg, 0.219 mmol) were added thereto. The reaction mixture was stirred at room temperature for 2 hours. Next, 0.6 mL of the activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (40 mg, 0.089 mmol) and DIPEA (0.047 mL, 0.268 mmol) in N,N-dimethylformamide (2.5 mL). The mixture was stirred at room temperature overnight. The reaction mixture was purified directly by acidic preparative MPLC (Luna10-50), and the product fractions were lyophilized to obtain a bright white solid.
Yield: 32 mg, 65%
U_AN_ACID: m/z 520.2 [M+H]⁺
¹H NMR (400 MHz, DMSO-d6) δ 8.33 (d, J = 8.7 Hz, 1H), 7.39 (s, 1H), 7.23 (s, 1H), 6.48 (s, 1H), 6.28 (d, J = 4.9 Hz, 2H), 5.50 (q, J = 6.7 Hz, 1H), 5.40 (s, 3H), 5.23 - 5.06 (m, 2H), 3.62 (d, J = 6.3 Hz, 1H), 3.03 (q, J = 6.2 Hz, 2H), 2.21 - 2.02 (m, 2H), 1.92 - 1.79 (m, 2H), 1.18 - 1.08 (m, 1H), 0.87 (t, J = 7.3 Hz, 3H), 0.47 - 0.30 (m, 4H).

### Preparation Example 8: Synthesis of Linker-Payload (mc-GGFG-PBX-7016) Compound

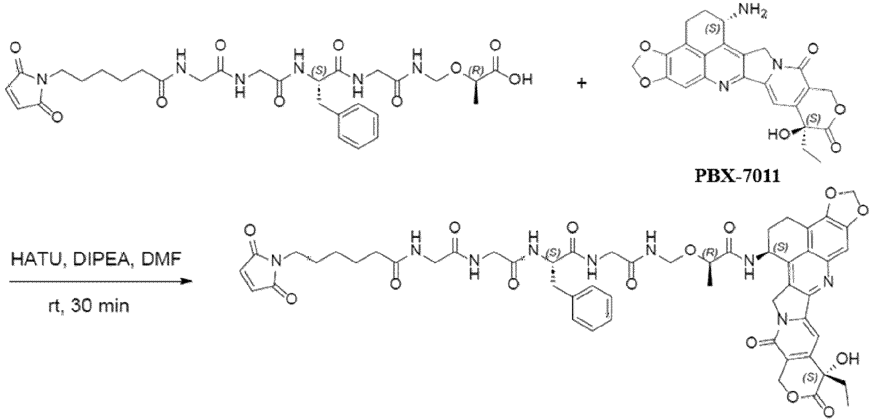

PBX-7011 (410 mg, 754 µmol) and (2R,10S)-10-benzyl-23-(2,5-dioxo-2,5-dihydro-1H-pyrrol-1-yl)-2-methyl-6,9,12,15,18-penta-oxo-3-oxa-5,8,11,14,17-pentaazatricosanoic acid (466 mg, 739 µmol), which has the mc-ggfg structure, were dissolved in 10 mL of N,N-dimethylformamide. DIPEA (439 mg, 3.39 mmol) and HATU (430 mg, 1.13 mmol) were dissolved successively and stirred at room temperature for 30 minutes. Then, the mixture was purified 3 times by acid preparative MPLC (Luna10-50), and the mc-GGFG-PBX-7016 compound was obtained as a light yellow solid through lyophilization.
Yield: 510 mg, 63%
U_AN_ACID: m/z 1060.02 [M+H]⁺
¹H NMR (400 MHz, DMSO-d6) δ 8.62 (t, J = 6.6 Hz, 1H), 8.50 (d, J = 9.1 Hz, 1H), 8.29 (t, J = 5.9 Hz, 1H), 8.13 - 7.96 (m, 3H), 7.39 (s, 1H), 7.27 - 7.12 (m, 6H), 6.99 (s, 2H), 6.48 (s, 1H), 6.26 (d, J = 6.0 Hz, 2H), 5.61 - 5.51 (m, 1H), 5.45 - 5.33 (m, 2H), 5.20 - 4.99 (m, 2H), 4.72 - 4.63 (m, 1H), 4.57 - 4.41 (m, 2H), 4.11 (q, J = 6.7 Hz, 1H), 3.78 - 3.53 (m, 6H), 3.40 - 3.34 (m, 2H), 3.16 - 2.95 (m, 3H), 2.74 (dd, J = 13.8, 9.5 Hz, 1H), 2.17 - 2.03 (m, 4H), 1.91 - 1.78 (m, 2H), 1.52 - 1.35 (m, 7H), 1.25 - 1.12 (m, 2H), 0.87 (t, J = 7.3 Hz, 3H).

### Example: Anti-TF ADC Including Formula 1 Compound (PBX Payload)

### Preparation of of Antibody-Drug Conjugates (ADCs)

Anti-TF ADCs, which include the PBX payload prepared in the Preparation Examples above, were prepared (Table 1).

**[Table 1]**

| No. | Payload | Linker | Antibody |
|---|---|---|---|
| Example 1 | PBX-7011 | | |
| Example 2 | PBX-7014 | | |
| Example 3 | PBX-7016 | | anti-TF antibody (Tisotumab, anti-TF mAb) |
| Example 4 | PBX-7018 | mc-GGFG | |
| Example 5 | PBX-7020 | | |
| Example 6 | PBX-7022 | | |
| Example 7 | PBX-7024 | | |

In the examples, linker-payloads, which include a PBX payload and into which GGFG (*i.e*., an enzymatically cleavable linker system) is introduced, were synthesized. Specifically, as the the linker-payload, those which have the molecular structure of an mc-GGFG-PBXPayload and a structure of Formula 13 as a linker were used. The linker was first reacted with the payload to create a linker-payload conjugate. For example, the linker-payloads included in Example 3 were synthesized by the method described in Preparation Example 8 above. Furthermore, ADCs were synthesized by conjugating with Tisotumab(Medchemexpress) as an anti-TF antibody. The ADCs were prepared by the following method. The prepared Tisotumab was buffer-exchanged with a reaction buffer (150 mM NaCl, 50 mM Histidine, pH 6.0) using a NAP-5 column (Cytiva) or PD-10 desalting column (Cytiva), and then treated the anitibody with 30 equivalents of 50 mM TCEP (Tris(2-carboxyethyl)phosphine hydrochloride) (Merck) at 25°C for 2 hours to generate thiol sites necessary for the reaction from the disulfide of the antibody.

Afterward excess TCEP was removed using a NAP-5 column or PD-10 desalting column, and was exchanged with reduction buffer (25 mM Histidine, pH 6.0). The conjugation reaction was performed by reacting 12 equivalents of the 5 mM linker-payload compounds prepared above with reduced Tisotumab at 4°C for 1 hour. As a result, Tisotumab-PBX-7016 (anti-TF-PBX-7016) ADCs were prepared in which linker-payload compounds were conjugated to an anti-TF antibody (anti-TF mAb).

### Evaluation of Antibody-Drug Conjugates (ADCs)

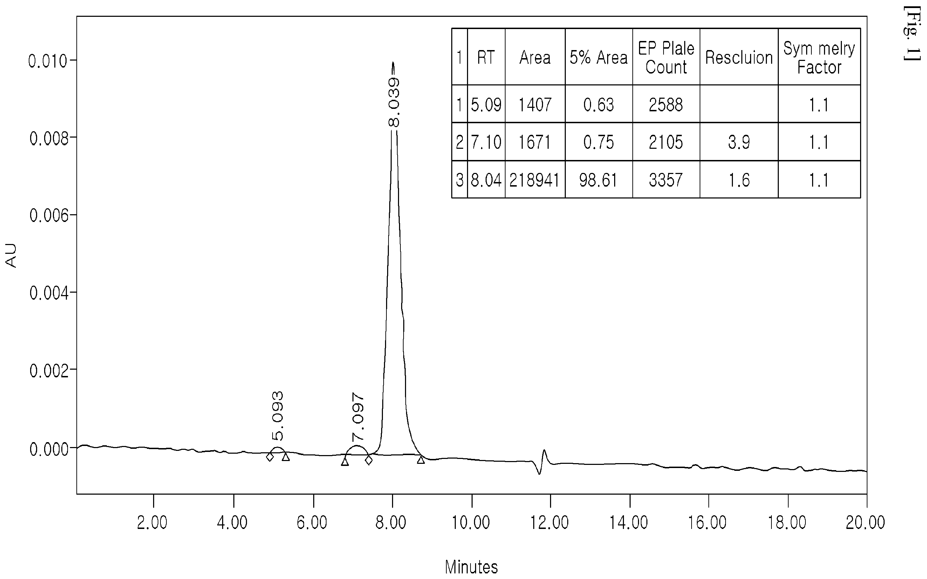

The purity of the ADC monomer of Example 3 was determined using size exclusion chromatography (SEC) as shown in Fig. 1 (2× PBS, 1 mg/mL, 280 nm).

The DAR was evaluated by the analysis of mass (Da) and area via LC-MS and the results are shown in Table 2. The SEC value was >98%.

**[Table 2]**

| Example 3 | | Mass (Da) | Area | Ratio | | DAR |
|---|---|---|---|---|---|---|
| Light Chain | D0 | 23375.5 | 51097.7 | 7.1 | 0.9 | |
| | D1 | 24435.4 | 669995.8 | 92.9 | | |
| | D2 | - | - | 0.0 | | |
| Heavy Chain | D0 | 50277.1 | - | 0.0 | 3.0 | 7.9 |
| | D1 | 51337.1 | - | 0.0 | | |
| | D2 | 52397.1 | - | 0.0 | | |
| | D3 | 53457.1 | 270006.1 | 100.0 | | |

### Comparative Example: Preparation of Isotype ADC and Anti-TF ADC with a different Payload

ADCs, in which an anti-influenza A antibody (Navivumab, P23) and an anti-SARS-CoV-2 antibody (Regdanvimab, P59) are each conjugated with PBX-7016 as a payload, were prepared using the methods described in the Examples above. P23 and P59 were used as isotype controls for this experiment. Meanwhile, Tivdak^{™} was purchased from Jupiter Research Service LLC and used.

Finally, ADCs, in which an anti-influenza A antibody (Navivumab, P23) was conjugated with monomethyl auristatin E (MMAE) as a payload, were prepared using the methods described in the Examples above. The anti-influenza A antibody (P23) is an antibody, which includes a heavy chain having an amino acid sequence of SEQ ID NO: 11 and a light chain having an amino acid sequence of SEQ ID NO: 12.

MMAE was purchased from MedChemExpress in the form of a payload and a linker-payload (MC-vc-PAB-MMAE). The ADCs with PBX-7016 as a payload were used in the form of a linker-payload (mc-GGFG-PBX-7016) using mc-GGFG as a linker.

The SEC and DAR values for each ADC above are as shown in Table 3 below.

**[Table 3]**

| | **ADC** | **SEC** | **DAR** |
|---|---|---|---|
| **1** | P23-PBX-7016 | >98% | 8 |
| **2** | P59-PBX-7016 | >98% | 8 |
| **3** | Tivdak (Tisotumab-MMAE) | >98% | 4 |
| **4** | P23-MMAE | >98% | 4 |

### Experimental Example 1: Cell Viability Assay of PBX Payload Anti-TF ADC

### Experimental Example 1-1. Selection of TF Expressing Cell Lines

Cell lines expressing Tissue factor (TF) were selected using flow cytometry.

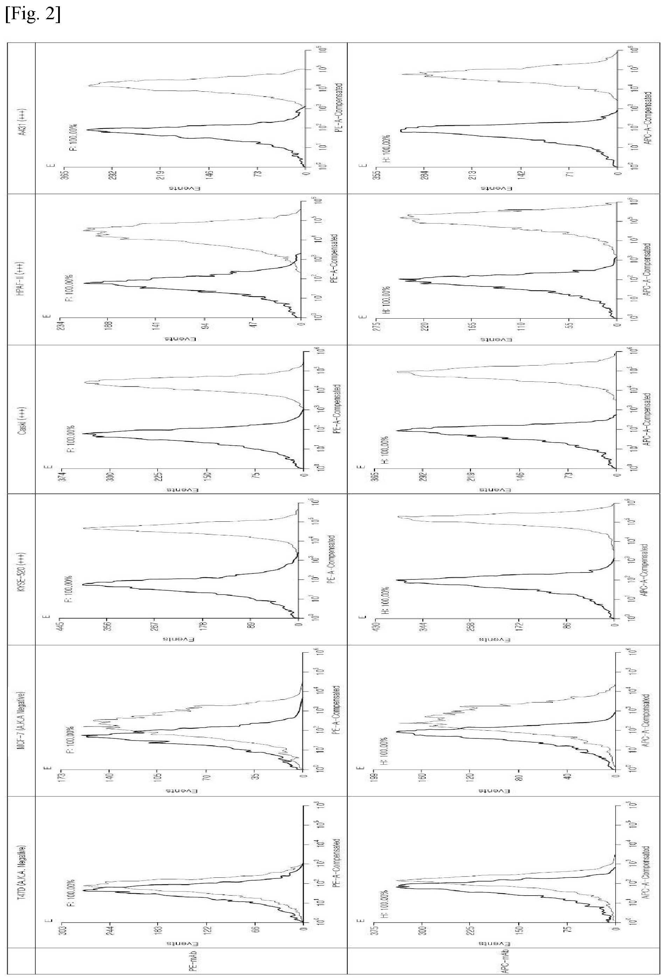

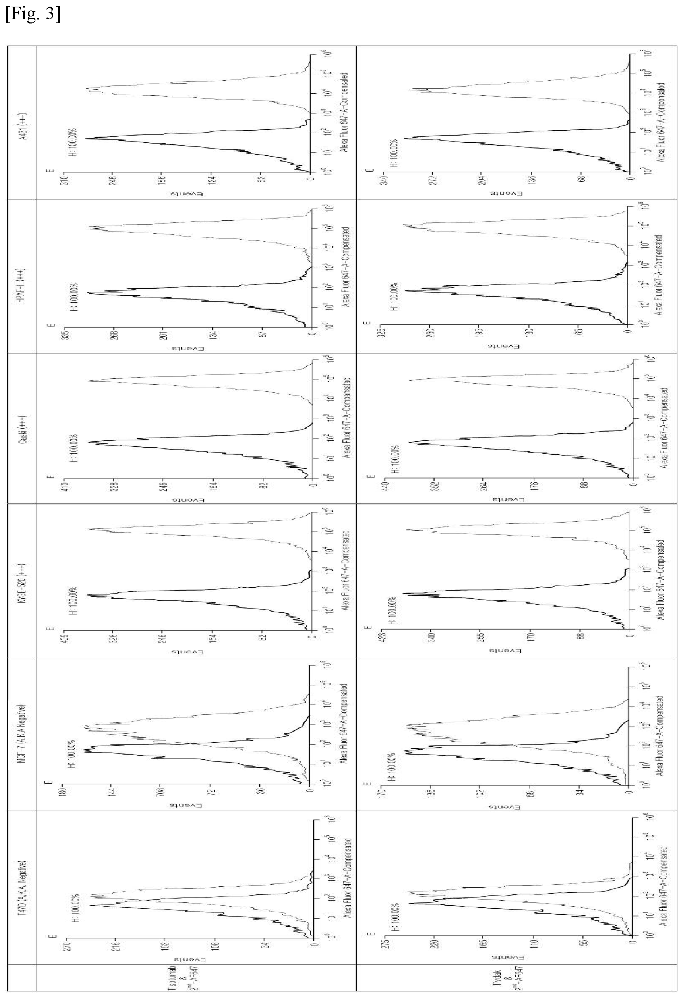

The cell lines used were T47D (a breast cancer cell line), MCF-7 (a breast cancer cell line), HPAF-II (a pancreatic cancer cell line), KYSE-520 (an esophageal cancer cell line), Caski (a cervical cancer cell line), and A431 (a skin cancer cell line). Antibody binding affinity and flow cytometry analyses were performed using commercially available monoclonal antibodies, PE-mAb (12-1429-42) and APC-mAb (365205) (see Fig. 2), and antibody binding affinity and flow cytometry analyses were performed using Tisotumab & AF647-F(ab)₂ anti-human IgG-F(ab)₂ (109-606-006) and Tivdak^{™} (Lot:196169) & AF647-F(ab)₂ anti-human IgG-F(ab)₂ (109-606-006) (see Fig. 3).

High levels of Tissue factor (TF) expression were observed in HPAF-II (a pancreatic cancer cell line), KYSE-520 (an esophageal cancer cell line), Caski (a cervical cancer cell line), and A431 (a skin cancer cell line), and the mean fluorescence intensity (MFI) and the staining index for each cell line are as shown in Table 4 below.

**[Table 4]**

| **Cell Line** | **Tissue (Cancer Type)** | **MFI** | | **Staining Index** | **Expression** |
|---|---|---|---|---|---|
| | | **No Staining** | **Conjugated mAb** | | |
| **T47D** | Breast | 45 | 92 | 2 | Negative or (+) |
| **MCF-7** | Breast | 53 | 515 | 10 | Negative or (+) |
| **KYSE-520** | Esophagus | 64 | 56,067 | 876 | +++ |
| **Caski** | Cervix | 49 | 29,318 | 598 | +++ |
| **HPAF-II** | Pancreas | 76 | 33,610 | 442 | +++ |
| **A431** | Skin | 78 | 21,016 | 269 | +++ |

### Experimental Example 1-2. Cell Viability Assay

An *in-vitro* cell viability assay was performed on the cell lines selected in Experimental Example 1-1 using the ADCs prepared in Example 3 and Comparative Example above, PBX-7016 payload compound, and MMAE payload compound.

Specifically, 1,000 to 3,000 cell lines listed in Table 3 were seeded per well in a 96-well plate and cultured at constant temperature (37°C, 5% CO₂). After 24 hours, the cells were treated with 100 µL of the payload compound or ADC drugs at 9 concentrations (a 4-fold serial dilution starting from 1,000 nM). In particular, a control group (drug concentration 0), which is an untreated group, was also prepared. After 6 days of incubation at 37°C and 5% CO₂, 100 µL of CellTiter-Glo reagent (CellTiter-Glo^{®} Luminescent Cell Viability Assay kit (Promega, G7571)) was added to each well and pipetted. Fluorescence was measured after incubation at room temperature (RT) for 10 minutes. The concentration that showed 50% of the fluorescence value when the drug concentration was 0 was considered 100%, and the concentration that showed 50% fluorescence value was measured as the IC₅₀ value.

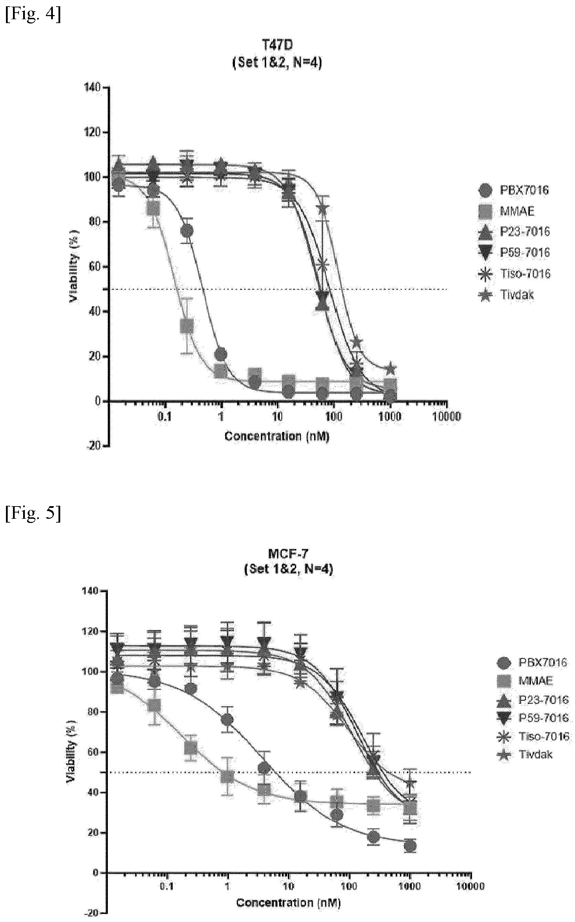

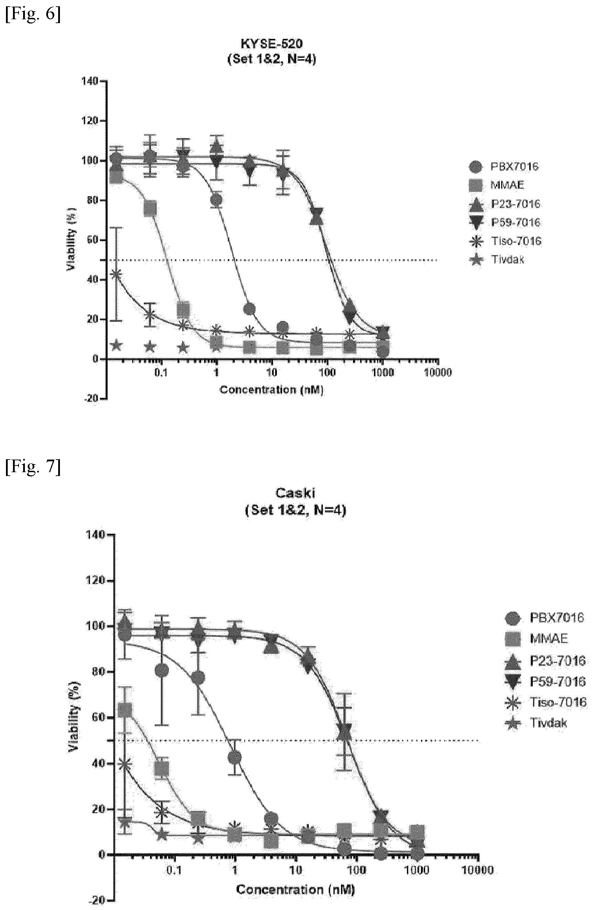

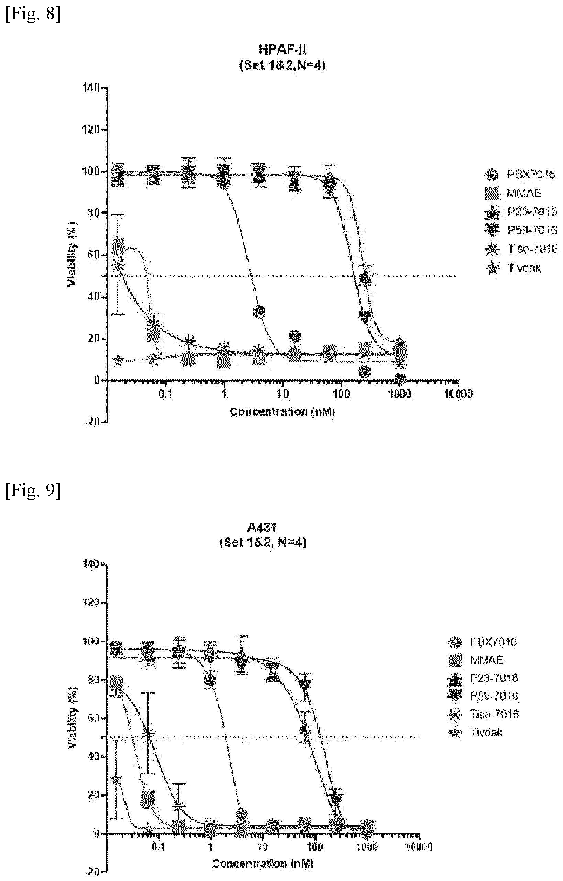

As a result, it was confirmed that the ADC of Example 3 (Tisotumab-PBX-7016) had excellent cell inhibition ability. The specific IC₅₀ values (unit: nM) for each ADC are as shown in Table 5 below (see Figs. 4 to 9).

**[Table 5]**

| **Cell Line** | **PBX-7016** | **MMAE** | **P23-PBX-7016 (Isotype ADC)** | **P59-PBX-7016 (Isotype ADC)** | **Tisotumab-PBX-7016 (Example 3)** | **Tivdak** |
|---|---|---|---|---|---|---|
| **T47D** | 0.46 | 0.16 | 55.2 | 57.3 | 84 | 137 |
| **MCF-7** | 6.02 | 0.82 | 256 | 294 | 357 | 442 |
| **KYSE-520** | 2.06 | 0.12 | 115 | 104 | < 0.015 | < 0.015 |
| **Caski** | 0.75 | 0.034 | 69.5 | 68.5 | < 0.015 | < 0.015 |
| **HPAF-II** | 2.87 | 0.045 | 251 | 165 | 0.018 | < 0.015 |
| **A431** | 1.94 | 0.032 | 71.5 | 126.5 | 0.065 | < 0.015 |

Specifically, in HPAF-II (a pancreatic cancer cell line), KYSE-520 (an esophageal cancer cell line), Caski (a cervical cancer cell line), and A431 (a skin cancer cell line), which are high-expressing cell lines in which Tissue factor (TF) is expressed in large amounts, it was observed that the ADC of Example 3 had a superior IC₅₀ value compared to the PBX-7016 compound itself or the isotype ADCs (P23-PBX-7016, P59-PBX-7016). Therefore, it was confirmed that when PBX-7016 was developed as an ADC, it could exhibit excellent inhibitory effects specifically against target cell lines expressing a large amount of Tissue factor (TF). In addition, it was observed that the ADC of Example 3 has an IC₅₀ value comparable to Tivdak^{™}, thus confirming that it has an excellent inhibitory effect on the target cell line.

Accordingly, the Tisotumab-PBX-7016 ADC of the present invention, which has a camptothecin derivative compound of Formula 1 as a payload and is specific for Tissue factor (TF), exhibits an excellent inhibitory effect on cells expressing Tissue factor (TF), and thus can be used for the treatment or prevention of cancer.

### Experimental Example 2: In vivo antitumor efficacy evaluation (1)

### Experimental Example 2-1 Confirmation of anticancer effect in KYSE520, which is human esophageal cancer xenograft model

### Experimental design

In order to evaluate the antitumor efficacy using KYSE520 (*i.e*., a human esophageal cancer xenograft model of BALB/c nude strain mice), the experiment was designed as follows (Table 6). Eighty-seven female mice, aged 6-8 weeks and weighing 18-20 g, plus 33 extra mice, were prepared.

**[Table 6]**

| **Group** | **Mouse Populatio n (N)** | **Treatment** | **Administ ration Dose (mg/kg)** | **Administr ation Volume (mL/kg)** | **Administra tion Route** | **Schedule** |
|---|---|---|---|---|---|---|
| G1 | 10 | Isotype mAb (P23) | 10 | 5 | *i.v.* | QW x 3 weeks |
| G2 | 7 | Tisotumab (Anti-TF mAb) | 10 | 5 | *i.v.* | QW x 3 weeks |
| G3 | 10 | Isotype-PBX-7016 | 10 | 5 | *i.v.* | QW x 3 weeks |
| G4 | 10 | Tivdak | 1 | 5 | *i.v.* | QW x 3 weeks |
| G5 | 10 | Tivdak | 3 | 5 | *i.v.* | QW x 3 weeks |
| G6 | 10 | Tivdak | 10 | 5 | *i.v.* | QW x 3 weeks |
| G7 | 10 | Tisotumab-PBX-7016 | 1 | 5 | *i.v.* | QW x 3 weeks |
| G8 | 10 | Tisotumab-PBX-7016 | 3 | 5 | *i.v.* | QW x 3 weeks |
| G9 | 10 | Tisotumab-PBX-7016 | 10 | 5 | *i.v.* | QW x 3 weeks |

Specifically, in Table 6 above, the isotype mAb (P23) is an anti-influenza A mAb called Navibumab, and the isotype-PBX-7016 is an ADC (P23-PBX-7016), in which P23 is used as an isotypye control. Meanwhile, the Tisotumab-PBX-7016 ADC has a structure in which Tisotumab (anti-TF mAb) and PBX-7016 are conjugated by a mc-GGFG linker, and Tivdak^{™} is an ADC in which MMAE is conjugated to Tisotumab.

### Cell Culture

KYSE520 tumor cells were maintained *in vitro* in RPMI 1640 medium supplemented with 10% fetal bovine serum and 1% antibiotic-antimycotic at 37°C under 5% CO₂ in air. Tumor cells were regularly passaged twice a week by trypsin-EDTA treatment, and cells growing in the exponential growth phase were harvested and counted for tumor inoculation.

### Tumor inoculation and administration of test substances

KYSE520 tumor cells (5 × 10⁶ cells/mouse) were subcutaneously inoculated into the right lateral side of each mouse with 0.2 mL of PBS and Matrigel (a 1:1 mixture). The animals were randomly assigned, and treatment was initiated on day 10 after tumor inoculation, when the mean tumor volume reached 175 mm³. The start date of treatment was set as day 0, and the animals were assigned to groups using Excel-based randomization software that performed hierarchical randomization according to tumor volume. Each group consisted of 10 or 7 tumor-bearing mice. The mice were administered with the test substances according to the regimens shown in Table 6 above.

### Tumor Measurement and Statistical Analysis

The primary purpose was to determine whether it could delay tumor growth or cure mice. The tumor volume was measured in two dimensions twice a week using calipers, and volume (mm³) was expressed using the following equation: $V = 0.5 a \times {b}^{2}$ (a: a long diameter of tumor, b: a short diameter of tumor)

The tumor size was used to calculate the ratio of T/C (tumor volume to control volume) and TGI (tumor growth inhibition) values. The T/C value (%) represents the antitumor effect, where T and C are the mean volumes of the treatment group and the control group, respectively, on a given day, and the T/C value (%) was calculated by dividing the tumor volume of the treatment group by the tumor volume of the vehicle group on the same day. The TGI of each group was calculated using the following equation: $TGI \left(%\right) = \left[1-\left({T}_{i}-{T}_{0}\right)/\left({V}_{i}-{V}_{0}\right)\right] \times 100 %$ (Ti: a mean tumor volume of the treatment group on a specific day, T0: a mean tumor volume of the treatment group on the first day of treatment, Vi: a mean tumor volume of the control group on the same day as Ti, V0: a mean tumor volume of the control group on the first day of treatment)

The mean and standard error of the mean (including SEM) at each time point for tumor volume in each group were determined. Statistical analysis was performed on the data obtained on day 35 after the start of the experiment to determine differences in tumor volume and tumor weight between groups. After performing the Kruskal-Wallis test, Dunn's *post hoc* analysis was performed to compare tumor volume and tumor weight. All data were analyzed using SPSS 29.0 (*p* < 0.05 was considered statistically significant).

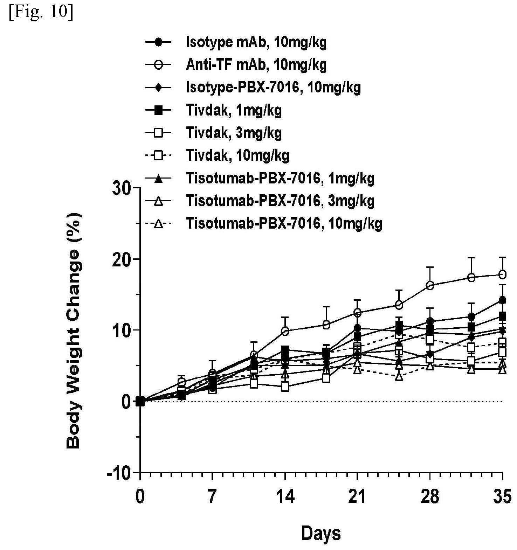

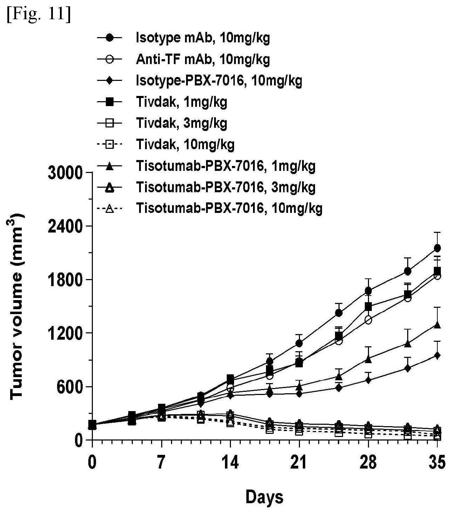

Animal body weights were regularly monitored as an indirect measure of toxicity. The body weight change (body weight change %) and tumor growth (tumor volume, mm³) curves by group are shown in Figs. 10 and 11. The mean tumor volume over time is shown in Table 7, and the TGI was calculated based on the tumor volume measurement on day 35 after the start of treatment and the results are shown in Table 8. T/C_{weight} was calculated based on the tumor weight measurement on day 35 after the start of treatment and the results are shown in Table 9.

**[Table 7]**

| Days^{b} | Tumor Volume (mm³)^{a} | | | | | | | | |
|---|---|---|---|---|---|---|---|---|---|
| | G1 | G2 | G3 | G4 | G5 | G6 | G7 | G8 | G9 |
| 0 | 175 ± 8 | 175 ± 10 | 175 ± 8 | 175 ± 8 | 175 ± 8 | 175 ± 8 | 175 ± 8 | 175 ± 8 | 175 ± 8 |
| 4 | 274 ± 16 | 270 ± 25 | 250 ± 11 | 279 ± 20 | 241 ± 23 | 243 ± 22 | 252 ± 17 | 224 ± 14 | 231 ± 12 |
| 7 | 362 ± 31 | 329 ± 31 | 319 ± 13 | 360 ± 29 | 279 ± 24 | 259 ± 24 | 353 ± 26 | 279 ± 22 | 273 ± 19 |
| 11 | 502 ± 42 | 448 ± 47 | 410 ± 22 | 489 ± 37 | 281 ± 31 | 234 ± 23 | 454 ± 38 | 291 ± 20 | 248 ± 14 |
| 14 | 680 ± 53 | 589 ± 62 | 505 ± 30 | 672 ± 51 | 270 ± 31 | 196 ± 16 | 534 ± 48 | 296 ± 31 | 213 ± 12 |
| 18 | 883 ± 82 | 731 ± 93 | 519 ± 41 | 771 ± 64 | 174 ± 17 | 122 ± 11 | 575 ± 59 | 203 ± 16 | 143 ± 6 |
| 21 | 1,083 ± 98 | 878 ± 111 | 521 ± 42 | 865 ± 75 | 154 ± 17 (n = 9)^{c} | 101 ± 7 | 606 ± 65 | 185 ± 16 | 130 ± 7 |
| 25 | 1,425 ± 109 | 1,116 ± 142 | 589 ± 54 | 1,168 ± 101 | 142 ± 15(n = 9) | 88 ± 7 | 718 ± 83 | 174 ± 16 | 123 ± 8 |
| 28 | 1,675 ± 135 | 1,347 ± 158 | 675 ± 86 | 1,497 ± 121 | 129 ± 16 (n = 9) | 69 ± 5 | 917 ± 125 | 161 ± 16 | 111 ± 8 |
| 32 | 1,895 ± 149 | 1,597 ± 148 | 810 ± 116 | 1,634 ± 126 | 115 ± 16(n = 9) | 58 ± 5 | 1,086 ± 158 | 145 ± 15 | 98 ± 7 |
| 35 | 2,151 ± 176 | 1,847 ± 175 | 947 ± 161 | 1,893 ± 166 | 99 ± 14 (n = 9) | 45 ± 6 | 1,298 ± 192 | 125 ± 12 | 61 ± 4 |

| | | | | | | | | | |
|---|---|---|---|---|---|---|---|---|---|
| a. mean ± SEM b. days after start of treatment c. On day 18, one mouse was sacrificed when its body weight was decreased by more than 20%. The average tumor volume was calculated from the surviving mice. | | | | | | | | | |

**[Table 8]**

| Group | Mouse Population (N) | Tumor Volume (mm³)^{a} on Day 35 | T/C^{b} (%) | TGI^{c} (%) | *p* Value^{d} |
|---|---|---|---|---|---|
| G1 | 10 | 2,151 ± 176 | - | - | - |
| G2 | 7 | 1,847 ± 175 | 85.86 | 15.40 | >0.999 |
| G3 | 10 | 947 ± 161 | 44.05 | 60.92 | >0.999 |
| G4 | 10 | 1,893 ± 166 | 88.03 | 13.03 | >0.999 |
| G5 | 9^{e} | 99 ± 14 | 4.58 | 103.89 | <0.001 |
| G6 | 10 | 45 ± 6 | 2.08 | 106.62 | <0.001 |
| G7 | 10 | 1,298 ± 192 | 60.36 | 43.16 | >0.999 |
| G8 | 10 | 125 ± 12 | 5.82 | 102.54 | 0.007 |
| G9 | 10 | 61 ± 4 | 2.83 | 105.80 | <0.001 |

| | | | | | |
|---|---|---|---|---|---|
| a. mean ± SEM b. T/C (%) = T/C × 100% (T and C are each the mean volume on day 35 after treatment in the treatment group and control group.) c. $TGI \left(%\right) = \left[1-\left({T}_{35}-{T}_{0}\right)/\left({V}_{35}-{V}_{0}\right)\right] \times 100 %$ d. *p* Values were calculated based on tumor volume. e. On day 18, one mouse was sacrificed due to a weight loss of more than 20%. | | | | | |

**[Table 9]**

| Group | Mouse Population (N) | Tumor Weights (g)^{a} on Day 35 | T/C_{weight}^{b} (%) | *p* Value^{c} |
|---|---|---|---|---|
| G1 | 10 | 2.157 ± 0.205 | - | - |
| G2 | 7 | 1.767 ± 0.224 | 81.94 | >0.999 |
| G3 | 10 | 0.987 ± 0.159 | 45.74 | >0.999 |
| G4 | 10 | 1.869 ± 0.138 | 86.67 | >0.999 |
| G5 | 9^{d} | 0.089 ± 0.013 | 4.12 | 0.002 |
| G6 | 10 | 0.042 ± 0.004 | 1.93 | <0.001 |
| G7 | 10 | 1.383 ± 0.205 | 64.12 | >0.999 |
| G8 | 10 | 0.106 ± 0.013 | 4.90 | 0.005 |
| G9 | 10 | 0.048 ± 0.005 | 2.22 | <0.001 |

| | | | | |
|---|---|---|---|---|
| a. mean ± SEM b. T/C (%) = T/C × 100% (T and C are each the mean volume on day 35 after treatment in the treatment group and control group.) c. *p* Values were calculated based on tumor weight. d. One mouse was sacrificed on day 18 when its body weight was decreased by more than 20%. | | | | |

All test substances of the tested dosage regimens were confirmed to be safe in female BALB/c nude mice implanted with KYSE520. On day 18 after the start of treatment, one mouse in G5 had a body weight loss of more than 20%, and was autopsied, but no abnormalities were found.

As shown in Tables 8 to 10, the mean tumor volume of G1 reached 2,151 mm³ on day 35 after the start of administration. Compared to G1, G5 (T/C = 4.58%, TGI = 103.89%, *p* < 0.001), G6 (T/C = 2.08%, TGI = 106.62%, *p* < 0.001), G8 (T/C = 5.82%, TGI = 102.54%, *p* = 0.007), and G9 (T/C = 2.83%, TGI = 105.80%, *p* < 0.001) showed statistically significant antitumor activity with the mean tumor volumes of 99 mm³, 45 mm³, 125 mm³, and 61 mm³, respectively, on day 35 after the start of treatment. Therefore, it can be seen that the antibody-drug conjugate (ADC) of the present invention exhibits excellent tumor growth inhibitory activity.

### Experimental Example 3: In vivo antitumor efficacy evaluation (2)

### Experimental Example 3-1. Confirmation of anticancer effect in HPAFII, which is human pancreatic cancer xenograft model

### Experimental design

In order to evaluate the antitumor efficacy using HPAFII (*i.e.,* a human pancreatic cancer xenograft model of CB17 SCID strain mice), the experiment was designed as follows (Table 10). One hundred twenty female mice, aged 6-8 weeks and weighing 17-22 g, plus 60 extra mice, were prepared.

**[Table 10]**

| Group | Mouse Population (N) | Treatment | Administr ation Dose (mg/kg) | Administrat ion Volume (mL/kg) | Administrat ion Route | Schedule |
|---|---|---|---|---|---|---|
| G1 | 10 | Isotype mAb (P23) | 3 | 5 | *i.v.* | QW × 3 |
| G2 | 10 | Tisotumab (Anti-TF mAb) | 3 | 5 | *i.v.* | QW × 3 |
| G3 | 10 | Isotype-MMAE | 3 | 5 | *i.v.* | QW × 3 |
| G4 | 10 | Tivdak | 0.3 | 5 | *i.v.* | QW × 3 |
| G5 | 10 | Tivdak | 1 | 5 | *i.v.* | QW × 3 |
| G6 | 10 | Tivdak | 1 | 5 | *i.v.* | QW × 1 |
| G7 | 10 | Tivdak | 3 | 5 | *i.v.* | QW × 1 |
| G8 | 10 | Isotype-PBX-7016 | 3 | 5 | *i.v.* | QW × 3 |
| G9 | 10 | Tisotumab-PBX-7016 | 0.3 | 5 | *i.v.* | QW × 3 |
| G10 | 10 | Tisotumab-PBX-7016 | 1 | 5 | *i.v.* | QW × 3 |
| G11 | 10 | Tisotumab-PBX-7016 | 1 | 5 | *i.v.* | QW × 1 |
| G12 | 10 | Tisotumab-PBX-7016 | 3 | 5 | *i.v.* | QW × 1 |

Specifically, in Table 10 above, the isotype mAb (P23) is an anti-influenza A mAb called Navivumab, and the isotype-PBX-7016 is an ADC (P23-PBX-7016), in which P23 is used as an isotypye control. Meanwhile, the Tisotumab-PBX-7016 ADC has a structure in which Tisotumab (anti-TF mAb) and PBX-7016 are conjugated by a mc-GGFG linker, and Tivdak^{™} is an ADC in which MMAE is conjugated to Tisotumab.

### Cell Culture

HPAFII tumor cells were maintained *in vitro* in EMEM medium supplemented with 10% fetal bovine serum and 1% antibiotic-antimycotic at 37°C under 5% CO₂ in air. Tumor cells were regularly passaged twice a week by trypsin-EDTA treatment, and cells growing in the exponential growth phase were harvested and counted for tumor inoculation.

### Tumor inoculation and administration of test substances

HPAFII tumor cells (3 × 10⁶ cells/mouse) were subcutaneously inoculated into the right lateral side of each mouse with 0.2 mL of PBS and Matrigel (a 1:1 mixture). The animals were randomly assigned and treatment was initiated day 9 after tumor inoculation, when the mean tumor volume reached 235 mm³. The start date of treatment was set as day 0, and the animals were assigned to groups using Excel-based randomization software that performed hierarchical randomization according to tumor volume. Each group consisted of 10 tumor-bearing mice. The mice were administered with the test substances according to the regimens shown in Table 10 above.

### Tumor Measurement and Statistical Analysis

Tumor measurements and statistical analyses were performed using data obtained on day 28 after the start of treatment.

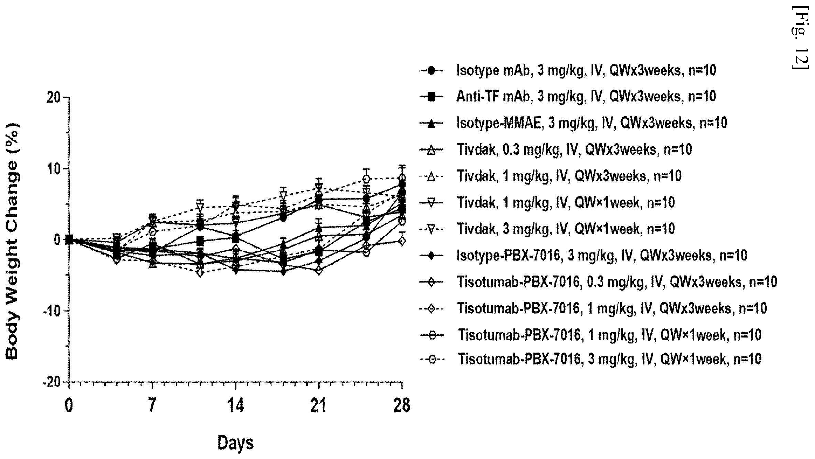

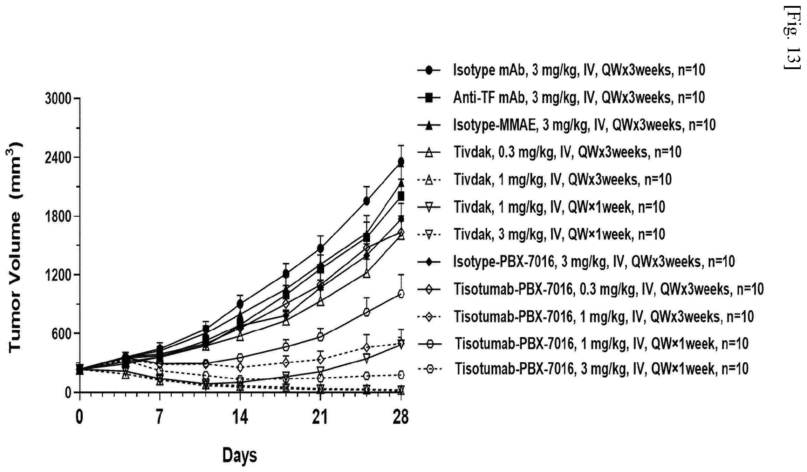

As an indirect measure of toxicity, animal body weights were regularly monitored, and the body weight change (body weight change %) and tumor growth (tumor volume, mm³) curves by group are shown in Figs. 12 and 13. The mean tumor volume over time is shown in Tables 11 and 12, and the TGI was calculated based on the tumor volume measurement on day 28 after the start of treatment and the results are shown in Table 13. T/C_{weight} was calculated based on the tumor weight measurement on day 28 after the start of treatment and the results are shown in Table 14.

**[Table 11]**

| Days^{b} | Tumor Volume (mm³)^{a} | | | | | |
|---|---|---|---|---|---|---|
| | G1 | G2 | G3 | G4 | G5 | G6 |
| 0 | 235 ± 17 | 235 ± 17 | 235 ± 17 | 235 ± 17 | 235 ± 17 | 235 ± 17 |
| 4 | 359 ± 50 | 290 ± 24 | 357 ± 38 | 313 ± 34 | 190 ± 12 | 217 ± 16 |
| 7 | 443 ± 64 | 362 ± 38 | 424 ± 46 | 361 ± 49 | 124 ± 11 | 141 ± 16 |
| 11 | 646 ± 78 | 527 ± 53 | 603 ± 79 | 476 ± 64 | 85 ± 9 | 87 ± 9 |
| 14 | 903 ± 90 | 690 ± 57 | 799 ± 98 | 576 ± 69 | 70 ± 8 | 103 ± 14 |
| 18 | 1,209 ± 105 | 996 ± 96 | 1,055 ± 125 | 737 ± 91 | 54 ± 6 | 161 ± 31 |
| 21 | 1,470 ± 125 | 1,262 ± 138 | 1,306 ± 147 | 934 ± 113 | 40 ± 4 | 213 ± 34 |
| 25 | 1,955 ± 145 | 1,581 ± 159 | 1,626 ± 182 | 1,219 ± 141 | 33 ± 4 | 344 ± 49 |
| 28 | 2,354 ± 165 | 2,004 ± 172 | 2,142 ± 164 | 1,607 ± 144 | 26 ± 3 | 486 ± 73 |

| | | | | | | |
|---|---|---|---|---|---|---|
| a. mean ± SEM b. days after start of treatment | | | | | | |

**[Table 12]**

| Days^{b} | Tumor Volume (mm³)^{a} | | | | | |
|---|---|---|---|---|---|---|
| | G7 | G8 | G9 | G10 | G11 | G12 |
| 0 | 235 ± 17 | 235 ± 17 | 235 ± 17 | 235 ± 17 | 235 ± 17 | 235 ± 17 |
| 4 | 216 ± 19 | 352 ± 41 | 362 ± 38 | 342 ± 30 | 355 ± 32 | 326 ± 31 |
| 7 | 128 ± 10 | 371 ± 41 | 389 ± 39 | 285 ± 31 | 295 ± 25 | 221 ± 26 |
| 11 | 69 ± 8 | 481 ± 46 | 498 ± 52 | 287 ± 43 | 295 ± 26 | 173 ± 20 |
| 14 | 56 ± 4 | 682 ± 59 | 657 ± 73 | 254 ± 43 | 354 ± 44 | 133 ± 12 |
| 18 | 39 ± 3 | 786 ± 56 | 906 ± 124 | 303 ± 69 | 464 ± 73 | 140 ± 13 |
| 21 | 30 ± 2 | 1,071 ± 77 | 1,098 ± 129 | 337 ± 83 | 564 ± 90 | 147 ± 15 |
| 25 | 26 ± 2 | 1,397 ± 119 | 1,474 ± 143 | 460 ± 129 | 819 ± 146 | 168 ± 26 |
| 28 | 19 ± 1 | 1,772 ± 157 | 1,636 ± 165 | 499 ± 146 | 1,007 ± 195 | 180 ± 35 |

| | | | | | | |
|---|---|---|---|---|---|---|
| a. mean ± SEM b. days after start of treatment | | | | | | |

**[Table 13]**

| Group | Mouse Population (N) | Tumor Volume (mm³)^{a} on Day 28 | T/C^{b} (%) | TGI^{c} (%) | *p* Value^{d} |
|---|---|---|---|---|---|
| G1 | 10 | 2,354 ± 165 | - | - | <0.001 |
| G2 | 10 | 2,004 ± 172 | 85.14 | 16.51 | |
| G3 | 10 | 2,142 ± 164 | 90.99 | 10.01 | |
| G4 | 10 | 1,607 ± 144 | 68.29 | 35.24 | |
| G5 | 10 | 26 ± 3 | 1.09 | 109.91 | |
| G6 | 10 | 486 ± 73 | 20.67 | 88.15 | |
| G7 | 10 | 19 ± 1 | 0.80 | 110.22 | |
| G8 | 10 | 1,772 ± 157 | 75.31 | 27.44 | |
| G9 | 10 | 1,636 ± 165 | 69.53 | 33.86 | |
| G10 | 10 | 499 ± 146 | 21.18 | 87.58 | |
| G11 | 10 | 1,007 ± 195 | 42.78 | 63.58 | |
| G12 | 10 | 180 ± 35 | 7.64 | 102.63 | |

| | | | | | |
|---|---|---|---|---|---|
| a. mean ± SEM b. T/C (%) = T/C × 100% (T and C are each the mean volume on day 28 after treatment in the treatment group and control group.) c. $TGI \left(%\right) = \left[1-\left({T}_{28}-{T}_{0}\right)/\left({V}_{28}-{V}_{0}\right)\right] \times 100 %$ d. *p* Values were calculated based on tumor volume. | | | | | |

**[Table 14]**

| Group | Mouse Population (N) | Tumor Weights (g)^{a} on Day 28 | T/C_{weight}^{b} (%) | *p* Value^{c} |
|---|---|---|---|---|
| G1 | 10 | 2.137 ± 0.144 | - | <0.001 |
| G2 | 10 | 1.856 ± 0.148 | 86.85 | |
| G3 | 10 | 1.933 ± 0.177 | 90.45 | |
| G4 | 10 | 1.461 ± 0.146 | 68.36 | |
| G5 | 10 | 0.022 ± 0.003 | 1.03 | |
| G6 | 10 | 0.454 ± 0.088 | 21.23 | |
| G7 | 10 | 0.012 ± 0.001 | 0.54 | |
| G8 | 10 | 1.810 ± 0.137 | 84.70 | |
| G9 | 10 | 1.691 ± 0.118 | 79.15 | |
| G10 | 10 | 0.449 ± 0.138 | 21.01 | |
| G11 | 10 | 1.070 ± 0.238 | 50.05 | |
| G12 | 10 | 0.144 ± 0.036 | 6.72 | |

| | | | | |
|---|---|---|---|---|
| a. mean ± SEM b. T/C (%) = T/C × 100% (T and C are each the mean volume on day 28 after treatment in the treatment group and control group.) c. *p* Values were calculated based on tumor volume. | | | | |

All test substances of the tested dosage regimens were confirmed to be safe in female CB17 SCID mice implanted with HPAFII. No significant weight loss was observed during the experiment.

As shown in Tables 11 to 14, the mean tumor volume of G1 reached 2,354 mm³ on day 28 after the start of administration. Compared to G1, G5 (T/C = 1.09%, TGI = 109.91%), G6 (T/C = 20.67%, TGI = 88.15%), G7 (T/C = 0.80%, TGI = 110.22%), G10 (T/C = 21.18%, TGI = 87.58%), and G12 (T/C = 7.64%, TGI = 102.63%) showed are statistically significant antitumor activity with the mean tumor volumes of 26 mm³, 486 mm³, 19 mm³, 499 mm³, and 180 mm³, respectively, on day 28 after the start of treatment. The results of tumor weight were consistent with those of tumor volume. Therefore, it can be seen that the antibody-drug conjugate (ADC) of the present invention exhibits excellent tumor growth inhibitory activity.

### Sequence Listing

The sequence listing of the antibodies used in the present invention is as described in Table 15 below.

**[Table 15]**

| SEQ ID NO: | Name of Sequence | Sequence |
|---|---|---|
| 1 | Anti-TF mAb CDR-H1 | GFTFSNYA |
| 2 | Anti-TF mAb CDR-H2 | ISGSGDYT |
| 3 | Anti-TF mAb CDR-H3 | ARSPWGYYLDS |
| 4 | Anti-TF mAb CDR-L1 | QGISSR |
| 5 | Anti-TF mAb CDR-L2 | AAS |
| 6 | Anti-TF mAb CDR-L3 | QQYNSYPYT |
| 7 | Anti-TF mAb heavy chain variable region | EVQLLESGGGLVQPGGSLRLSCAASGFTFSNYAMSWVRQAPGK GLEWVSSISGSGDYTYYTDSVKGRFTISRDNSKNTLYLQMNSLR AEDTAVYYCARSPWGYYLDSWGQGTLVTVSS |
| 8 | Anti-TF mAb light chain variable region | DIQMTQSPPSLSASAGDRVTITCRASQGISSRLAWYQQKPEKAPK SLIYAASSLQSGVPSRFSGSGSGTDFTLTISSLQPEDFATYYCQQY NSYPYTFGQGTKLEIK |
| 9 | Anti-TF mAb heavy chain | EVQLLESGGGLVQPGGSLRLSCAASGFTFSNYAMSWVRQAPGK GLEWVSSISGSGDYTYYTDSVKGRFTISRDNSKNTLYLQMNSLR AEDTAVYYCARSPWGYYLDSWGQGTLVTVSSASTKGPSVFPLA PSSKSTSGGTAALGCLVKDYFPEPVTVSWNSGALTSGVHTFPAVL QSSGLYSLSSVVTVPSSSLGTQTYICNVNHKPSNTKVDKKVEPKS CDKTHTCPPCPAPELLGGPSVFLFPPKPKDTLMISRTPEVTCVVV DVSHEDPEVKFNWYVDGVEVHNAKTKPREEQYNSTYRVVSVLT VLHQDWLNGKEYKCKVSNKALPAPIEKTISKAKGQPREPQVYTL PPSRDELTKNQVSLTCLVKGFYPSDIAVEWESNGQPENNYKTTPP |
| | | VLDSDGSFFLYSKLTVDKSRWQQGNVFSCSVMHEALHNHYTQK SLSLSPGK |
| 10 | Anti-TF mAb light chain | DIQMTQSPPSLSASAGDRVTITCRASQGISSRLAWYQQKPEKAPK SLIYAASSLQSGVPSRFSGSGSGTDFTLTISSLQPEDFATYYCQQY NSYPYTFGQGTKLEIKRTVAAPSVFIFPPSDEQLKSGTASVVCLLN NFYPREAKVQWKVDNALQSGNSQESVTEQDSKDSTYSLSSTLTL SKADYEKHKVYACEVTHQGLSSPVTKSFNRGEC |
| 11 | Isotype mAb (P23) heavy chain | MDWTWSILFLVAAATGAHSQVQLVQSGAEVKKPGASVKVSCKT SGYSFSTYGVSWVRQAPGQGPEWVGWISAYTGITDYAQKFQGR VTLTTDATTATAFLDLRSLRPDDTATYFCARDKVQGRVEVGSGG RHDYWGQGTLVIVSSASTKGPSVFPLAPSSKSTSGGTAALGCLV KDYFPEPVTVSWNSGALTSGVHTFPAVLQSSGLYSLSSVVTVPSS SLGTQTYICNVNHKPSNTKVDKKVEPKSCDKTHTCPPCPAPELL GGPSVFLFPPKPKDTLMISRTPEVTCVVVDVSHEDPEVKFNWYV DGVEVHNAKTKPREEQYNSTYRVVSVLTVLHQDWLNGKEYKC KVSNKALPAPIEKTISKAKGQPREPQVYTLPPSRDELTKNQVSLT CLVKGFYPSDIAVEWESNGQPENNYKTTPPVLDSDGSFFLYSKLT VDKSRWQQGNVFSCSVMHEGLHNHYTQKSLSLSPGK* |
| 12 | Isotype mAb (P23) light chain | METETPAQLLFLLLLWLPDTTGEVVLTQSPGTLALPPGERATLSCRA SHRVGSTYIAWYQQKSGQAPRRLIYGASNRATDIPDRFSGSGSGT DFTLTIRRLEPEDSAVYYCQQFSVSPWTFGQGTRVEIKRTVAAPS VFIFPPSDEQLKSGTASVVCLLNNFYPREAKVQWKVDNALQSGN SQESVTEQDSKDSTYSLSSTLTLSKADYEKHKVYACEVTHQGLS SPVTKSFNRGEC |

## Claims

1. An antibody-drug conjugate (ADC) comprising a structure, in which a compound according to Formula 1 below or a pharmaceutically acceptable salt, a stereoisomer, a tautomer, a prodrug, a hydrate, a solvate, or an isotopically labeled analog thereof is linked to an anti-Tissue factor antibody via a linker: 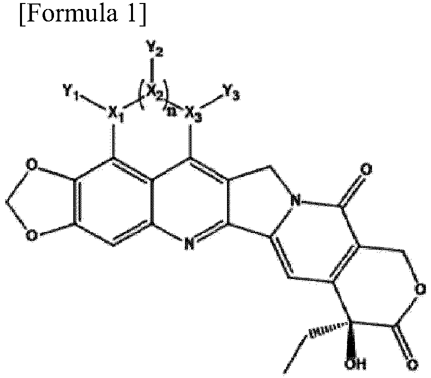 wherein:
X₁ and X₃ are each independently carbon, oxygen, nitrogen, or sulfur, and X₁ and X₃ are the same or different;
X₂ is carbon, oxygen, nitrogen, sulfur, a single bond, or a double bond;
n is 0, 1, or 2; and
Y₁, Y₂, and Y₃ are each independently hydrogen or a functional group comprising oxygen, nitrogen, phosphorus, or sulfur.

2. The antibody-drug conjugate of claim 1, wherein:
X_{1,} X₂, and X₃ are each independently carbon;
n is 1;
Y₁ and Y₂ are each independently hydrogen;
Y₃ is -NZ₁Z₂; and
Z₁ and Z₂ each comprise a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³; and
wherein R¹, R², and R³ are each independently hydrogen, hydroxyl, C₁₋₃ alkyl, C₃₋₇ cycloalkyl, or C₁₋₃ haloalkyl.

3. The antibody-drug conjugate of claim 1, wherein:
X_{1,} X₂, and X₃ are each independently carbon;
n is 1;
Y₁ and Y₂ are each independently hydrogen;
Y₃ is -NZ₁Z₂; and
Z₁ is hydrogen, and Z₂ is C(=O)(C₂₋₃ alkenyl) or C(=O)CR¹R²R³, wherein R¹, R², and R³ are each independently hydrogen, hydroxyl, C₁₋₃ alkyl, C₃₋₇ cycloalkyl, or C₁₋₃ haloalkyl.

4. The antibody-drug conjugate of claim 1, wherein the compound of Formula 1 is represented by any one of Formulas 2 to 11 below:

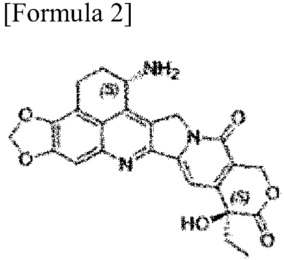

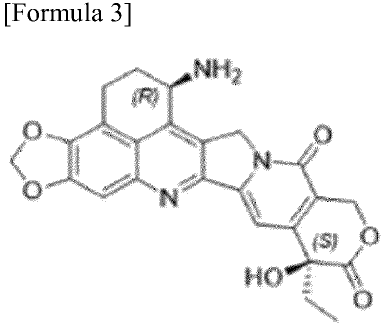

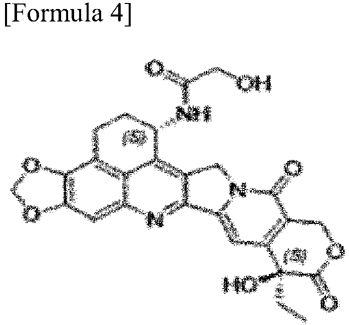

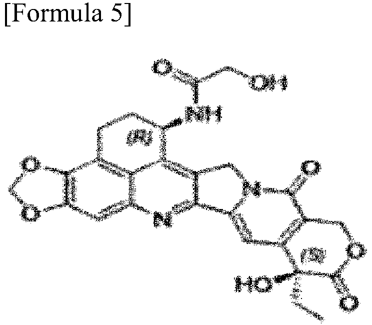

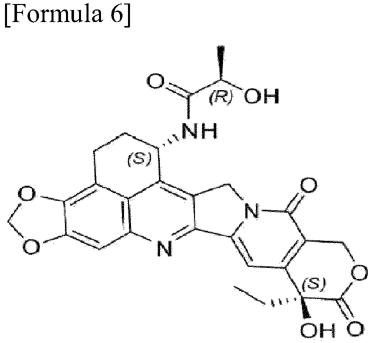

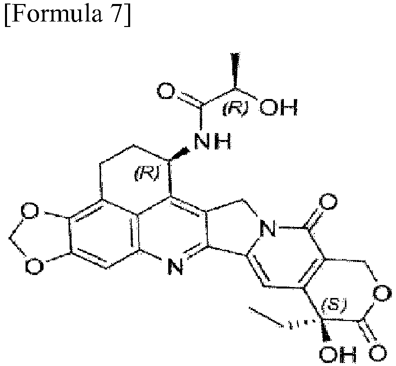

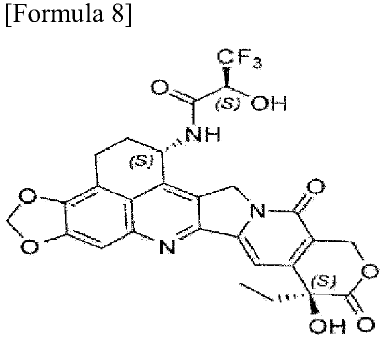

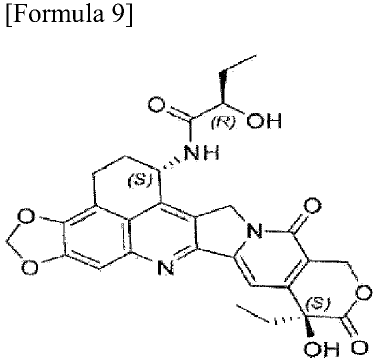

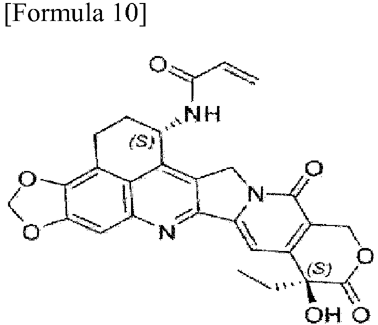

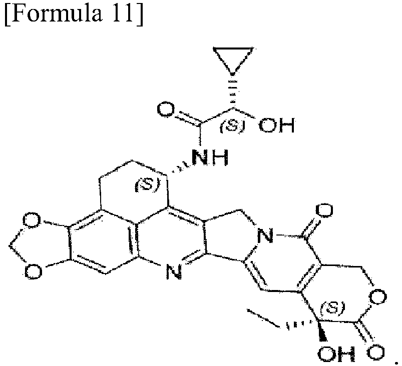

5. The antibody-drug conjugate (ADC) of claim 1, wherein the linker is represented by Formula 12 below: 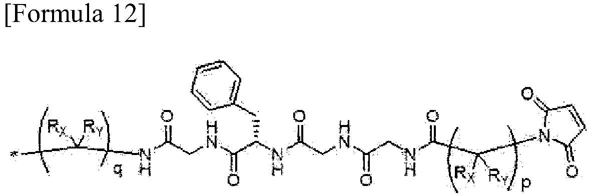 wherein:
R_{X} and R_{Y} are each independently hydrogen or C₁₋₆ alkyl;
p and q are each independently an integer of 1 to 6; and
* is the part that binds to a payload.

6. The antibody-drug conjugate of claim 1, wherein the anti-tissue factor antibody is selected from the group consisting of antibodies of Tisotumab, XB-002, MRG-004A, AMB101, and STRO-004.

7. The antibody-drug conjugate of claim 1, wherein the anti-Tissue factor antibody is Tisotumab.

8. The antibody-drug conjugate of claim 1, wherein the anti-Tissue factor antibody is an antibody comprising i) a heavy chain comprising CDR-H1, CDR-H2, and CDR-H3 having the amino acid sequences of SEQ ID NOs: 1, 2 and 3, respectively, and ii) a light chain comprising CDR-L1, CDR-L2, and CDR-L3 having the amino acid sequences of SEQ ID NOs: 4, 5, and 6, respectively; or an antigen-binding fragment thereof.

9. The antibody-drug conjugate of claim 1, wherein the anti-Tissue factor antibody is an antibody comprising i) a heavy chain variable region having the amino acid sequence of SEQ ID NO: 7, and ii) a light chain variable region having the amino acid sequence of SEQ ID NO: 8; or an antigen-binding fragment thereof.

10. The antibody-drug conjugate of claim 1, wherein the anti-Tissue factor antibody is an antibody comprising i) a heavy chain having the amino acid sequence of SEQ ID NO: 9, and ii) a light chain having the amino acid sequence of SEQ ID NO: 10; or an antigen-binding fragment thereof.

11. A pharmaceutical composition for treating or preventing cancer, comprising the antibody-drug conjugate according to any one of claims 1 to 10 as an active ingredient.

12. The pharmaceutical composition of claim 11, wherein the cancer is selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, fallopian tube cancer, ovarian epithelial cancer, ovarian germ cell cancer, male breast cancer, brain cancer, pituitary adenoma, multiple myeloma, gallbladder cancer, biliary tract cancer, colorectal cancer, head and neck cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, paranasal sinus and nasal cavity cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, uretheral cancer, cancer of unknown primary site, gastric lymphoma, gastric cancer, gastric carcinoid, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi's sarcoma, Paget's disease, tonsil cancer, squamous cell carcinoma, pulmonary adenocarcinoma, lung cancer, pulmonary squamous cell carcinoma, skin cancer, anal cancer, rhabdomyosarcoma, laryngeal cancer, pleural cancer, blood cancer, and thymic cancer.

13. The pharmaceutical composition of claim 11, wherein the cancer is selected from the group consisting of cervical cancer, endometrial cancer, ovarian cancer, peritoneal cancer, fallopian tube cancer, breast cancer, head and neck cancer, esophageal cancer, bladder cancer, and pancreatic cancer.

14. A method for treating or preventing cancer, comprising administering to a subject an antibody-drug conjugate according to any one of claims 1 to 10.

15. Use of the antibody-drug conjugate according to any one of claims 1 to 10 for treating or preventing cancer.

16. Use of the antibody-drug conjugate according to any one of claims 1 to 10 for preparing a pharmaceutical composition for treating or preventing cancer.
